# EUROPEAN PATENT SPECIFICATION

(11) **EP 2 010 671 B1**
(45) Date of publication and mention of the grant of the patent: **18.01.2012**
(21) Application number: 06829636.7
(22) Date of filing: 14.12.2006
(51) Int. Cl.: C12Q 1/68

(54) **DETECTION AND QUANTIFICATION OF FAECAL POLLUTION IN AN ENVIRONMENTAL SAMPLE**
NACHWEIS UND QUANTIFIZIERUNG FÄKALER VERUNREINIGUNGEN IN UMWELTPROBEN
DÉTECTION ET QUANTIFICATION D'UNE POLLUTION FÉCALE DANS UN ÉCHANTILLON ENVIRONNEMENTAL

(30) Priority: 11.04.2006 AT 6252006; 11.04.2006 AT 6262006
(43) Date of publication of application: 07.01.2009
(73) Proprietor: Technische Universität Wien, 1040 Wien (AT)
(72) Inventor: REISCHER, Georg, A-1140 Wien (AT); FARNLEITNER, Andreas, A-2632 Wimpassing (AT); MACH, Robert, A-3011 Untertullnerbach (AT); KASPER, David, A-1110 Wien (AT)
(74) Representative: Krauss, Jan
(86) International application number: PCT/EP2006/012086
(87) International publication number: WO 2007/115590

(56) References cited:
- WO-A-02/27014
- REISCHER GEORG H ET AL: "Quantitative PCR method for sensitive detection of ruminant fecal pollution in freshwater and evaluation of this method in alpine karstic regions." APPLIED AND ENVIRONMENTAL MICROBIOLOGY AUG 2006, vol. 72, no. 8, August 2006 (2006-08), pages 5610-5614, XP002428212 ISSN: 0099-2240
- LAYTON ALICE ET AL: "Development of Bacteroides 16S rRNA gene TaqMan-based real-time PCR assays for estimation of total, human, and bovine fecal pollution in water." APPLIED AND ENVIRONMENTAL MICROBIOLOGY JUN 2006, vol. 72, no. 6, June 2006 (2006-06), pages 4214-4224, XP002428213 ISSN: 0099-2240
- SEURINCK SYLVIE ET AL: "Detection and quantification of the human-specific HF183 Bacteroides 16S rRNA genetic marker with real-time PCR for assessment of human faecal pollution in freshwater." ENVIRONMENTAL MICROBIOLOGY FEB 2005, vol. 7, no. 2, February 2005 (2005-02), pages 249-259, XP002428205 ISSN: 1462-2912 cited in the application
- DICK LINDA K ET AL: "Rapid estimation of numbers of fecal Bacteroidetes by use of a quantitative PCR assay for 16S rRNA genes." APPLIED AND ENVIRONMENTAL MICROBIOLOGY SEP 2004, vol. 70, no. 9, September 2004 (2004-09), pages 5695-5697, XP002428204 ISSN: 0099-2240 cited in the application
- DICK LINDA K ET AL: "Host distributions of uncultivated fecal Bacteroidales bacteria reveal genetic markers for fecal source identification." APPLIED AND ENVIRONMENTAL MICROBIOLOGY JUN 2005, vol. 71, no. 6, June 2005 (2005-06), pages 3184-3191, XP002428208 ISSN: 0099-2240 cited in the application
- KREADER C A: "Design and evaluation of Bacteroides DNA probes for the specific detection of human fecal pollution." APPLIED AND ENVIRONMENTAL MICROBIOLOGY APR 1995, vol. 61, no. 4, April 1995 (1995-04), pages 1171-1179, XP002428207 ISSN: 0099-2240 cited in the application
- FOGARTY LISA R ET AL: "Comparison of bacteroides-prevotella 16S rRNA genetic markers for fecal samples from different animal species." APPLIED AND ENVIRONMENTAL MICROBIOLOGY OCT 2005, vol. 71, no. 10, October 2005 (2005-10), pages 5999-6007, XP002428209 ISSN: 0099-2240 cited in the application
- BERNHARD A E ET AL: "A PCR assay To discriminate human and ruminant feces on the basis of host differences in Bacteroides-Prevotella genes encoding 16S rRNA." APPLIED AND ENVIRONMENTAL MICROBIOLOGY OCT 2000, vol. 66, no. 10, October 2000 (2000-10), pages 4571-4574, XP002428206 ISSN: 0099-2240 cited in the application
- OTT STEPHAN J ET AL: "Quantification of intestinal bacterial populations by real-time PCR with a universal primer set and minor groove binder probes: a global approach to the enteric flora." JOURNAL OF CLINICAL MICROBIOLOGY JUN 2004, vol. 42, no. 6, June 2004 (2004-06), pages 2566-2572, XP002428203 ISSN: 0095-1137
- AFONINA I A ET AL: "MINOR GROOVE BINDER-CONJUGATED DNA PROBES FOR QUANTITATIVE DNA DETECTION BY HYBRIDIZATION-TRIGGERED FLUORESCENCE" BIOTECHNIQUES, INFORMA LIFE SCIENCES PUBLISHING, WESTBOROUGH, MA, US, vol. 32, no. 4, April 2002 (2002-04), pages 940-944,946, XP001087262 ISSN: 0736-6205

## Description

The present invention relates to oligonucleotides, uses thereof and methods for the detection and/or quantification of faecal pollution in environmental samples, especially of human and/or ruminant faecal pollution, as well as for the discrimination between human and ruminant faecal pollution in environmental samples. The oligonucleotides of the present invention can be used to specifically target a human and/or a ruminant marker sequence. The oligonucleotides are therefore especially useful as primers and/or probes in a method comprising a real time PCR. The invention can be applied in the field of microbial source tracking (MST) and in quantitative microbial risk assessment (QMRA) studies as part of water safety plans.

### BACKGROUND OF THE INVENTION

Groundwater resources from alpine and mountainous karst aquifers play an important role in public water supply throughout the world (Ford and Williams, 1996). Catchment protection and optimised abstraction are a requirement in the use of karstic spring water as a drinking water source resulting in a usually high raw water quality with no need for extensive treatment. Occasionally, however, karst springs show vulnerability to faecal contamination from point sources like leaking septic systems and non-point sources like wildlife and grazing livestock (Sinton et al., 1998).

The fact that alpine regions serve various purposes often leads to conflicts between stakeholders in tourism, hunting and forestry on the one side and the drinking water installations on the other side. The identification and apportioning of the sources of faecal contamination will make management and mitigation of this problem much easier and cost-effective (Simpson et al., 2002).

Microbial source tracking (MST) serves this purpose and makes it possible to pinpoint pollution sources. Microbial Source Tracking (MST), also referred to as bacterial source tracking (BST), is a method used to determine the sources of faecal microorganisms and establish whether faecal microorganisms (such as faecal bacteria) are being introduced into water bodies through human, wildlife, agricultural, or pet wastes. MST is considered to be a novel technology still in developmental stages. There are four categories of methods currently being developed and used in MST: biochemical (phenotype), molecular (genotype), chemical, and immunological.

The levels of faecal pollution in alpine karstic spring water range from no detectable pollution to quite high levels under unfavourable precipitation conditions and in vulnerable aquifers (Farnleitner et al., 2005). This wide range demands for a MST methodology with supreme sensitivity as well as adequate selectivity (Kralik, 2001). Currently no method is available meeting these requirements for MST in karstic spring water. In addition, MST aims at identifying the sources of faecal contamination in aquatic systems (Simpson et al., 2002). As a tool for the management of water sources used for drinking water production or recreation it pinpoints sources of faecal input and makes directed measures for catchment protection possible. Depending on the investigated catchment area potential sources range from humans (leaking septic systems, combined sewer overflow) to livestock and wildlife (surface run-off from grazing animals). In addition, the gained information about the origin of pollution facilitates the assessment of the potential risk posed by the presence of faecal microorganisms.

Despite the growing concerns about water transmissible zoonoses (Sinton et al., 1998), human specific pathogens like the hepatitis A virus or *Salmonella enterica* serovar Typhi are a main concern in drinking water hygiene (Scott et al., 2002). Tools which help to specifically rule out or quantify human faecal influence would be very helpful in risk assessment studies.

The group of dominant faecal anaerobes and in particular the phylum *Bacteroidetes* has been attracting the attention of the scientific MST community for some time now because of the relatively high abundance of these bacteria in faeces as compared to the usual faecal indicators such as *E. coli* or the faecal coliforms (Suau et al., 1999). Members of the group formerly named *Cytophaga*/*Flavobacter*/*Bacteroides* (CFB group) have been proposed as faecal indicator organisms (Allsop and Stickler, 1984, 1985; Fiksdal et al., 1985) and have been shown to exhibit host adaptation on the genetic level (Gordon, 2001; Dick et al., 2005). Developments in the field of molecular biology have led to methods circumventing the drawbacks of anaerobic cultivation techniques (Kreader, 1995, 1998; Dick and Field, 2004). More recently these bacteria became the target of source tracking efforts. Bernhard and Field (2000) identified human and cattle specific genetic markers and developed methods for their detection. These were subsequently applied in practice (Bernhard and Field, 2000; Bernhard et al., 2003; Boehm et al., 2003) and improved (Seurinck et al., 2005).

Quantitative real-time PCR (RTQ-PCR) assays have been demonstrated to be useful in the detection and quantification of microorganisms in the environment (Suzuki et al., 2000; Roe et al., 2001; Stults et al., 2001; Brinkman et al., 2003; Guy et al., 2003; Reischer et al., 2004). The probe-based quantitative PCR (qPCR) assays like the 5'-nuclease assays are among the most stable and reliable qPCR formats. They are preferable to assays using DNA-binding dyes like SYBR Green I because of the improved specificity introduced by the probe sequence (Bustin, 2000).

The present invention aims to improve the prior art and it is, thus, an object of the present invention to provide tools and uses thereof as well as methods for detecting and/or quantifying faecal pollution of human or ruminant origin. It is furthermore an object to provide tools and methods for discriminating human faecal pollution from ruminant faecal pollution.

### SUMMARY OF THE INVENTION

According to the present invention this object is solved by providing an oligonucleotide comprising a nucleic acid sequence selected from the group consisting of TCATGATCCCATCCTG (SEQ ID NO: 1), TCATGATGCCATCTTG (SEQ ID NO: 2), or a nucleic acid sequence complementary thereto. An oligonucleotide of the invention can furthermore comprise a minor groove binder (MGB), a locked nucleic acid (LNA) and/or a peptide nucleic acid (PNA) as well as at least one detectable molecule (label).

The object is further solved by the use of at least one oligonucleotide of the invention as a probe or a primer for the detection and/or quantification of faecal pollution in an environmental sample. An oligonucleotide of the invention can furthermore be used for discriminating between human and ruminant faecal pollution.

The object is further solved by a kit for the detection and/or quantification of faecal pollution in an environmental sample by real time PCR comprising at least one, preferably two oligonucleotides according to the present invention.

The object is further solved by a method for the detection and/or quantification of faecal pollution in an environmental sample, comprising subjecting an extracted nucleic acid from the environmental sample to real time PCR using at least two oligonucleotides according to the present invention, wherein one oligonucleotide is used as a primer and the other oligonucleotide is used as a probe. The method can further comprise a step of discriminating between human and ruminant faecal pollution.

The present invention and its preferred embodiments are described in further detail below.

### DESCRIPTION OF THE PREFERRED EMBODIMENTS OF THE INVENTION

It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

As outlined above, the present invention provides an oligonucleotide comprising a nucleic acid sequence selected from the group consisting of TCATGATCCCATCCTG (SEQ ID NO: 1), TCATGATGCCATCTTG (SEQ ID NO: 2).

The oligonucleotide according to the invention can further comprise a nucleic acid sequence selected from a group further consisting of TCATGATGCCATCCTG (SEQ ID NO: 8) or a nucleic acid sequence complementary thereto.

An "oligonucleotide" as used herein refers to the polymeric form of deoxyribonucleotides, ribonucleotides or modified nucleotides. Oligonucleotides of the present invention are typically up to 100 nucleotides, preferably up to 50 nucleotides, more preferably up to 30 and even more preferably up to 20 nucleotides in length. Most preferred are the oligonucleotides selected from SEQ ID NOs: 1 to 2 or 8. Furthermore, an oligonucleotide of the present invention is an isolated oligonucleotide.

The oligonucleotides of the present disclosure can comprise or consist of truncated or elongated nucleic acid sequences of SEQ ID NOs: 1 to 7 or of SEQ ID NO: 8. The person of skill in the art is able to select the suitable oligonucleotides dependent on the respective application and assay.

Preferably, the oligonucleotide comprises a nucleic acid sequence selected from the group consisting of TCATGATCCCATCCTG (SEQ ID NO: 1), TCATGATGCCATCTTG (SEQ ID NO: 2), or a nucleic acid sequence complementary thereto.

It was found that specific sequences in the 16S rRNA gene of members of the bacterial group *Bacteroidetes* were dominant in human faeces and thus constituted ideal human markers for use with quantitative real-time PCR. The nucleic acid sequences with SEQ ID NOs: 1, 2, 4 and 5 and also the nucleic acid sequence with SEQ ID NO: 8 are derived from that human marker sequence and can, thus, specifically hybridize to that sequence. Preferred uses resulting therefrom are explained in further detail below and in the Examples.

It was furthermore found that other specific sequences in the 16S rRNA gene of members of the bacterial group *Bacteroidetes* were only present in ruminant faeces and thus constituted an ideal ruminant marker for use with quantitative real-time PCR. The nucleic acid sequences with SEQ ID NOs: 3, 6 and 7 are derived from that ruminant marker sequence and can, thus, specifically hybridize to that sequence. Preferred uses resulting therefrom are explained in further detail below and in the Examples.

Preferably, the oligonucleotide consists of a nucleic acid sequence selected from the group of SEQ ID NOs: 1 to 2 or 8 or a nucleic acid sequence complementary thereto.

In a preferred embodiment, the oligonucleotide according to the present invention further comprises a minor groove binder (MGB), a locked nucleic acid (LNA) and/or a peptide nucleic acid (PNA), preferably a MGB moiety.

A "minor groove binder" (MGB) moiety binds to the minor groove of DNA with high affinity. When this minor groove binder is conjugated to one end (the 5'-end or the 3'-end) of short oligodeoxynucleotides, the conjugates form unusually stable hybrids with complementary DNA in which the tethered MGB group resides in the minor groove.

A "locked nucleic acid" (LNA) is a RNA derivative in which the ribose ring is constrained by a methylene linkage between the 2'-oxygen and the 4'-carbon. This conformation restriction increases binding affinity for complementarity sequences.

Chimeric DNA-PNA (peptide nucleic acid) and pure PNA molecular beacon probes can be used to provide stronger binding of the qPCR probe. An unique property of the peptide nucleic acid (PNA) molecular beacon is that the stem-loop structure is not required for quenching when used in molecular beacon qPCR. The discriminatory advantage of PNA probes is further enhanced by their increased affinity for DNA. This allows the use of short PNA probes.

Preferably, an oligonucleotide comprising or containing nucleic acid sequences selected from SEQ ID NOs: 1 to 2 or 8 or a nucleic acid sequence complementary thereto comprises an MGB moiety, LNA or PNA, more preferably an MGB.

In a preferred embodiment, the oligonucleotide according to the present invention further comprises at least one, preferably two detectable molecules.

A "detectable molecule" or "label" is a molecule capable of detection. In one embodiment, the label is conjugated to a nucleotide sequence. For example the label can be directly conjugated to a nucleic acid sequence (or to a particular nucleoside triphosphate thereof) or can become bound thereto by being bound to a specific binding agent that is attached to a probe or primer nucleotide sequence. In general, any label that is detectable can be used. Examples of labels that can be used to practice the methods disclosed herein include, but are not limited to: isotopic or non-isotopic, catalysts such as an enzyme or a catalytic polynucleotide, promoter, dye, fluorescent molecule, chemiluminescent molecule, coenzyme, enzyme substrate, radioactive group, small organic molecule, amplifiable polynucleotide sequence, a particle such as latex or carbon, metal sol, crystallite, liposome, cell, etc., which may or may not be further labeled with a dye, catalyst, or other detectable group, and the like. In another embodiment labels include an oligonucleotide or specific polynucleotide sequence that can be added to a probe or primer to provide a template for amplification or ligation. In one embodiment, a detectable label is a member of a signal-producing system which generates a detectable signal alone or together with other members of the signal-producing system.

Preferred detectable molecules or labels according to the invention are selected from the group of chromophores, fluorophores, chemiluminescent molecules, isotopes, enzymes, coenzymes, antigens, antibodies and their fragments, substrates. A person of skill in the art will be capable of selecting suitable detectable molecules/labels depending on e.g. the mode of detection, the sensitivity required.

In a preferred embodiment the oligonucleotide according to the present invention comprises two detectable molecules, wherein one is a fluorescence donor or reporter and the other is a fluorescence quencher or acceptor. In such a case the two detectable molecules form a fluorescence resonance energy transfer (FRET) pair.

Fluorophores or dyes that are suitable FRET pairs are known in the art, such that the person of skill in the art will be able to select the most suited for the respective application. The fluorescence donor or reporter is preferably selected from FAM, VIC, JOE, HEX, TET, Cy4, Texas Red, ROX and others known in the art. The fluorescence quencher or acceptor is preferably selected from NFQ, TAMRA, BodyPi 564/570, Cy5, Black Hole Quencher 1 (BHQ-1), BHQ-2, BHQ-3 and others known in the art.

In a preferred embodiment the oligonucleotide according to the present invention comprises FAM as fluorescence donor or reporter and NFQ as the fluorescence quencher or acceptor.

In one preferred embodiment the oligonucleotide comprises an MGB, a non-fluorescent fluorescence quencher, such as NFQ, and a fluorescence donor, such as FAM. Preferably, the fluorescence quencher is attached to the 3' terminus of the oligonucleotide and the fluorescence donor is attached tot the 5' terminus of the oligonucleotide. More preferably, the fluorescence quencher is the linkage between the oligonucleotide and MGB.

Furthermore, at least one oligonucleotide according to the present disclosure is used as a primer for the detection and/or quantification of faecal pollution in an environmental sample, preferably an oligonucleotide that comprises or contains nucleic acid sequences selected from SEQ ID NOs: 4 to 7 or a nucleic acid sequence complementary thereto.

A "primer" as used herein is an oligonucleotide that is complementary to a given DNA sequence and that is needed to initiate replication by DNA polymerase. PCR primers are short fragments of single stranded nucleic acids, such as DNA (15-30 nucleotides in length) that are complementary to nucleic acid (DNA) sequences that flank the target region of interest. The purpose of PCR primers is to provide a "free" 3'-OH group to which the DNA polymerase can add dNTPs.

In the present invention, the oligonucleotides when used as primers specifically bind/anneal to the marker gene sequence, i.e. the human marker or the ruminant marker of faecal pollution, such that the marker gene sequence or parts thereof can be amplified, but only if present in the sample.

The following oligonucleotides are primers, wherein the respective SEQ ID NO of the nucleic acid sequence is shown in "[ ]":

| | | |
|---|---|---|
| BacH_f | CTTGGCCAGCCTTCTGAAAG | [SEQ ID NO: 4], |
| | ATCGTCTACCGAAAATAC | [SEQ ID NO: 5], |
| BacR_f | GCGTATCCAACCTTCCCG | [SEQ ID NO: 6], |
| | ATCCCCATCCGTTACCG | [SEQ ID NO: 7]. |

The following oligonucleotides are further primers:

| | |
|---|---|
| BacH_r | CCCCATCGTCTACCGAAAATAC |
| BacR_r | CATCCCCATCCGTTACCG |

### See also the Examples.

At least one oligonucleotide according to the present invention is preferably used as a probe for the detection and/or quantification of faecal pollution in an environmental sample, preferably an oligonucleotide that comprises or contains nucleic acid sequences selected from SEQ ID NOs: 1 to 2 or 8 or a nucleic acid sequence complementary thereto.

A "probe" as used herein is an oligonucleotide that specifically hybridizes to a target sequence, such as a marker gene, or to a part of said target, and that is preferably labelled with detectable molecules allowing the detection of the hybridization of the probe to its target, either afterwards or at real time. Probes are used to detect the complementary base sequence by hybridization.

In the present invention, the oligonucleotide when used as a probe specifically hybridizes to the marker gene sequence, i.e the human marker or the ruminant marker of faecal pollution. Preferably, the probe is designed to hybridize to the amplified marker gene sequence, allowing the monitoring of the PCR. Due to a preferred labelling with a fluorescent donor/reporter and a fluorescent quencher/acceptor, the hybridization of the probe can be detected and/or quantified, preferably by monitoring it in real time, when the marker gene sequence or its amplified product, respectively, is present.

The following oligonucleotides are disclosed, wherein the respective SEQ ID NO of the nucleic acid sequence is shown in "[ ]":

| | | |
|---|---|---|
| BacH_pC | FAM-5' -TCATGATCCCATCCTG-3' -NFQ-MGB | [SEQ ID NO: 1], |
| BacH_pT | FAM-5' -TCATGATGCCATCTTG-3' -NFQ-MGB | [SEQ ID NO: 2], |
| BacR_p | FAM-5' -CTTCCGAAAGGGAGATT-3' -NFQ-MGB | [SEQ ID NO: 3]. |

### See also the Examples.

Importantly, these oligonucleotides are highly suitable as probes, wherein BacH_pC and/or BacH_pT are suitable to probe for human faceal pollution and BacR_p is suitable to probe for ruminant faceal pollution (see Examples).

The following oligonucleotide is also preferred, wherein the respective SEQ ID NO of the nucleic acid sequence is shown in "[ ]":

| | | |
|---|---|---|
| BacH_p | FAM-5' -TCATGATGCCATCCTG-3' -NFQ-MGB | [SEQ ID NO: 8]. |

Also BacH_p is suitable to probe for human faceal pollution.

A preferred embodiment of the present invention is the use of the oligonucleotides in a real time PCR or quantitative real-time PCR (RTQ-PCR), respectively.

A "quantitative real-time PCR" (RTQ-PCR, also referred to as qPCR) or "real time PCR" system is based on the detection and quantitation of a fluorescent reporter, either in form of fluorescent dyes that intercalate with double-strand DNA, or modified DNA oligonucleotide probes that fluoresce when hybridized with a complementary DNA. The signal increases or decreases in direct proportion to the amount of PCR product in a reaction. By recording the amount of fluorescence emission at each cycle, it is possible to monitor the PCR reaction during exponential phase where the first significant increase in the amount of PCR product correlates to the initial amount of target template.

A "TaqMan PCR" is a 5' nuclease quantitative PCR system. It is based on the 5'-3' exonuclease activity of the Taq DNA polymerase, which results in cleavage of fluorescent dye-labelled probes during PCR; the intensity of fluorescence is then measured. The TaqMan probe is located between the two PCR primers and preferably has a melting temperature higher than that of the primers. Binding of the TaqMan probe prior to the primers is crucial because without it PCR products would be formed without generation of fluorescence intensity and thus without being detected. The TaqMan probe has two fluorescent tags attached to it. One is a reporter dye, such as 6-carboxyfluorescein (FAM), which has its emission spectra quenched due to the spatial proximity of a second fluorescent dye, such as 6-carboxy-tetramethyl-rhodamine (TAMRA). Degradation of the TaqMan probe, by the Taq DNA polymerase, frees the reporter dye from the quenching activity of e.g. TAMRA and thus the fluorescent activity increases with an increase in cleavage of the probe, which is proportional to the amount of PCR product formed.

The oligonucleotides are especially suitable for the detection and/or quantification of faecal pollution in an environmental sample, wherein said faecal pollution is human or ruminant faecal pollution.

Preferably, oligonucleotides comprising or containing nucleic acid sequences selected from SEQ ID NOs: 1 or 2 or 8 or a nucleic acid sequence complementary thereto are suitable as probes with respect to human faecal pollution.

The oligonucleotides, compositions, kits and methods of the present invention are especially suitable for the detection and/or quantification of faecal pollution in environmental samples. An environmental sample is selected from a water sample, a soil or sediment sample, a compost sample, a biofilm sample, plant material, a meat sample or a food product sample. The water samples are preferably taken from ground water, spring water, surface water, sea water, waste water, seepage water, drinking water, or groundwater bodies, springs, water distribution systems, rivers, lakes, marine environments, sewage and waste water treatments systems.

Furthermore, an environmental sample according to the present invention is preferably a pooled and not an individual human faecal sample. An environmental sample according to the present invention is preferably a pooled and not an individual ruminant faecal sample.

In a preferred embodiment of the present invention, the oligonucleotides are used for discriminating between human and ruminant faecal pollution.
There are different preferred possibilities to perform said discrimination. In one embodiment the sample or nucleic acid extracted from it is subjected to the oligonucleotides of the present invention wherein the oligonucleotides used as probes are suitably labelled with detectable molecules which can be distinguished from each other allowing for the detection and/or quantification of human and ruminant faecal pollution (i.e. the respective marker sequences) at the same time. In another preferred embodiment one aliquot of the sample or of the nucleic acid extracted from it is subjected to the oligonucleotides of the present invention specific for human faecal pollution and another aliquot of the sample or of the nucleic acid extracted from it is subjected to the oligonucleotides of the present invention specific for ruminant faecal pollution, each in a different reaction setup, such as a PCR or a real time PCR. A person of skill in the art will be able to design suitable reaction setups and choose suitable detectable molecules. Further preferred embodiments for discriminating between human and ruminant faecal pollution are described in more detail below (see methods of the present invention).

A preferred embodiment of the invention is a composition comprising at least one oligonucleotide according to the present invention.

Preferably, a composition suitable for the detection and/or quantification of human faecal pollution comprises oligonucleotides comprising or containing nucleic acid sequences selected from SEQ ID NOs: 1, 2 and/or 8 or a nucleic acid sequence complementary thereto. Also disclosed is that a composition suitable for the detection and/or quantification of ruminant faecal pollution comprises oligonucleotides comprising or containing nucleic acid sequences selected from SEQ ID NOs: 3, 6 and/or 7 or a nucleic acid sequence complementary thereto.

A composition can further comprise further means or agents necessary for performing a PCR, preferably a real time PCR, such as enzymes, buffer, nucleotides.

A preferred embodiment of the invention is a kit for the detection and/or quantification of faecal pollution in an environmental sample by real time PCR comprising at least one oligonucleotide according to the present invention.

The kit preferably further comprises primer designed by 16S rRNA gene sequence alignment, preferably the 16S rRNA of *Bacteroidetes.* Primer sequences are preferably derived from consensus sequences found in the sequence alignments.

At least one oligonucleotide of the kit is used as a primer and at least one other oligonucleotide of the kit is used as a probe in the PCR.

Preferably, the oligonucleotide used as a probe comprises or contains nucleic acid sequences selected from SEQ ID NOs 1 to 2 or 8 or a nucleic acid sequence complementary thereto.

As described above, oligonucleotides comprising or containing nucleic acid sequences selected from SEQ ID NOs: 1 or 2 or 8 or a nucleic acid sequence complementary thereto are suitable probes.

As described above, an oligonucleotide comprising or containing a nucleic acid sequence with SEQ ID NO: 3 or a nucleic acid sequence complementary thereto is a suitable probe and oligonucleotides comprising or containing nucleic acid sequences selected from SEQ ID NOs: 6 or 7 or a nucleic acid sequence complementary thereto are suitable primers with respect to ruminant faecal pollution.

In a preferred embodiment, the kit further comprises a quantification standard. Preferably, the quantification standard has a defined amount of marker equivalents and is applied in a defined dilution series. Preferably, this standard is a plasmid carrying the marker sequence or a purified solution of the specific PCR product of known concentration (see also Examples).

The kit is preferably applicable for the detection and/or quantification of human or ruminant faecal pollution.

As described above, the oligonucleotides of the present invention are suitable for discriminating between human and ruminant faecal pollution. Thus, the kit according to the present invention is suitable for said discrimination as well. A person of skill in the art will be able to choose the components of the kit.

A further preferred embodiment is a method for the detection and/or quantification of faecal pollution in an environmental sample. Such a method comprises detecting and/or quantifying at least one marker sequence. The method especially comprises the following steps:
a) providing an environmental sample,
b) extracting nucleic acid from said sample,
c) subjecting the extracted nucleic acid to real time PCR using at least two oligonucleotides according to the invention, wherein one is used as a primer and the other is used as a probe, or using a composition according to the invention, and optionally, using a quantification standard with a defined amount of marker equivalents, and
d) optionally, quantifying the marker equivalents of the sample.

As described above, two marker sequences for faecal pollution were determined, a marker sequence for human faecal pollution ("human marker sequence" or "human faecal marker") and a marker sequence for ruminant faecal pollution ("ruminant marker sequence" or "ruminant faecal marker"). The method utilizes these marker sequences. If at least one of the marker sequences is present in the sample it can be detected and/or quantified by the method.

The sample provided is preferably concentrated. Concentrating a sample can be performed by filtration, centrifugation or osmose or other techniques known in the art. Especially for water samples, concentrating is preferred. Preferred filters for water samples are polycarbonate filters.

The preferred method for extracting the nucleic acid from the environmental sample depends on the environmental sample, which can be a water sample as well as a soil or sediment sample. Chemical extraction is preferred. The person of skill in the art is familiar with nucleic acid extraction methods and is able to select a suitable method depending on the sample. For water samples chemical DNA extraction using phenol/chloroform/isoamylalcohol supplemented by bead-beating is a preferred extraction method.

Preferably the at least one oligonucleotide used as a probe in step c) is an oligonucleotide comprising or containing a nucleic acid sequence selected from SEQ ID NOs: 1 to 2 or 8 or a nucleic acid sequence complementary thereto.

The amount of marker sequence that has been amplified by the real time PCR of the method is preferably quantified. By using a quantification standard with a known/defined amount of the marker sequence (i.e. a defined amount of "marker equivalents"), which will also be amplified at the same time, the amount of the marker sequence that had been present (before amplification) can be determined. Thus, quantifying the marker equivalents of the sample of step d) refers to quantifying the original marker equivalents of the sample. The respective concentration of the "marker equivalents" in respect to the sample volume is referred to as "marker equivalent concentration".

The marker equivalents of the sample are preferably quantified by using regression curves obtained by using the quantification standard with a defined amount of marker equivalents.

Further methods for quantification are known in the art and include e.g. spiking, direct calculation from threshold data and serial dilution.

The method is suitable for the detection and/or quantification of faecal pollution, wherein said faecal pollution is human or ruminant faecal pollution.

As described above, oligonucleotides comprising or containing nucleic acid sequences selected from SEQ ID NOs: 1 or 2 or 8 or a nucleic acid sequence complementary thereto are suitable probes

The method of the present disclosure targets at least one marker sequence specific for human faecal pollution, wherein the method comprises the following steps:
a) providing an environmental sample, such as a water sample, and concentrating said sample,
b) extracting nucleic acid from said sample,
c) subjecting the extracted nucleic acid to quantitative real-time PCR using a primer pair and at least one oligonucleotide probe,
   wherein the reverse primer comprises the nucleic acid sequence of SEQ ID NO: 5 and the at least one oligonucleotide probe is selected from a probe having attached a minor groove binder (MGB) moiety and a probe containing locked nucleic acid (LNA) or peptide nucleic acid (PNA) nucleotides, and comprises a nucleic acid sequence selected from SEQ ID NO: 1, SEQ ID NO: 2 and the nucleic acids complementary thereto, and using at the same time a quantification standard with a defined amount of marker equivalents, and
d) quantifying the marker equivalents of the sample.

In case of a water sample, the sample is preferably concentrated by filtration.

The method of the present disclosure targets at least one marker sequence specific for ruminant faecal pollution, wherein the method comprises the following steps:
a) providing an environmental sample, such as a water sample, and
   concentrating said sample,
b) extracting nucleic acid from said sample,
c) subjecting the extracted nucleic acid to quantitative real-time PCR using a primer pair and an oligonucleotide probe,
   wherein the reverse primer comprises the nucleic acid sequence of SEQ ID NO: 7, and the oligonucleotide probe is selected from a probe having attached a minor groove binder (MGB) moiety and a probe containing locked nucleic acid (LNA) or peptide nucleic acid (PNA) nucleotides, and comprises the nucleic acid sequence of SEQ ID NO: 3 or the nucleic acid complementary thereto, and using at the same time a quantification standard with a defined amount of marker equivalents, and
d) quantifying the marker equivalents of the sample.

In case of a water sample, the sample is preferably concentrated by filtration.

The method of the present invention is preferably applied for discriminating between human and ruminant faecal pollution in an environmental sample. Thus, the method of the present invention further comprises a discrimination between human and ruminant faecal pollution.

Preferably, the extracted nucleic acid is divided in at least two aliquots in step b) and in step c) a first aliquot of the extracted nucleic acid is subjected to a first quantitative real-time PCR and a second aliquot of the extracted nucleic acid is subjected to a second quantitative real-time PCR, wherein one PCR uses oligonucleotides of the present invention specific for human faecal pollution and the other PCR uses oligonucleotides of the present invention specific for ruminant faecal pollution. Preferably the two quantitative real-time PCR reactions are carried out in parallel.

In embodiments where the method of the present invention is applied for discriminating between human and ruminant faecal pollution in an environmental sample at least one marker sequence specific for human faecal pollution and at least one marker sequence specific for ruminant faecal pollution is detected and/or quantified. Then the method preferably comprises the following steps:
a) providing an environmental sample,
b) extracting nucleic acid from said sample and dividing the extracted nucleic acid in at least two aliquots,
c) subjecting a first aliquot of extracted nucleic acid to a first quantitative real-time PCR and subjecting a second aliquot of extracted nucleic acid to a second quantitative real-time PCR using at least two oligonucleotides according to the invention for each quantitative real-time PCR, wherein in each case one is used as a primer and the other is used as a probe,
   or using a composition according to the invention for each quantitative real-time PCR, and optionally, using a quantification standard with a defined amount of marker equivalents, and
d) optionally, quantifying the marker equivalents of the sample.

Preferably, in the first PCR of step c) at least one oligonucleotide comprising or containing a nucleic acid sequence selected from SEQ ID NOs: 1 or 2 or 8 or a nucleic acid sequence complementary thereto is used as a probe for detecting and/or quantifying human faecal pollution.

Also described is that in the second PCR of step c) an oligonucleotide comprising or containing a nucleic acid sequence with SEQ ID NO: 3 or a nucleic acid sequence complementary thereto is used as a probe and oligonucleotides comprising or containing nucleic acid sequences selected from SEQ ID NOs: 6 or 7 or a nucleic acid sequence complementary thereto are used as primers for detecting and/or quantifying ruminant faecal pollution.

The method then comprises the following steps:
a) providing an environmental sample,
b) extracting nucleic acid from said sample and dividing the extracted nucleic acid in at least two aliquots,
c) subjecting a first aliquot of extracted nucleic acid to a first quantitative real-time PCR and subjecting a second aliquot of extracted nucleic acid to a second quantitative real-time PCR
   using a primer pair and an oligonucleotide probe for each quantitative real-time PCR, wherein in the first quantitative real-time PCR the reverse primer comprises the nucleic acid sequence of SEQ ID NO: 5,
   and the oligonucleotide probe is selected from a probe having attached a minor groove binder (MGB) moiety and a probe containing locked nucleic acid (LNA) or peptide nucleic acid (PNA) nucleotides, and comprises a nucleic acid sequence selected from of SEQ ID NO: 1, SEQ ID NO: 2, SEQ ID NO: 8 or the nucleic acid complementary thereto, and using at the same time a quantification standard with a defined amount of marker equivalents, and
   wherein in the second quantitative real-time PCR the reverse primer comprises the nucleic acid sequence of SEQ ID NO: 7,
   and the oligonucleotide probe is selected from a probe having attached a minor groove binder (MGB) moiety and a probe containing locked nucleic acid (LNA) or peptide nucleic acid (PNA) nucleotides, and comprises the nucleic acid sequence of SEQ ID NO: 3 or the nucleic acid complementary thereto, and using at the same time a quantification standard with a defined amount of marker equivalents, and d) quantifying the human marker equivalents and the ruminant marker equivalents of the sample.

Furthermore, it is also preferred to subject the extracted nucleic acid (of step b) in step c) to one quantitative real-time PCR, wherein oligonucleotides of the present invention specific for human faecal pollution and oligonucleotides of the present invention specific for ruminant faecal pollution are used in one reaction. The oligonucleotides used as probes preferably comprise detectable molecules that can be distinguished from each other and are all detected in real time. Suitable detectable molecules are for instance fluorophores that emit fluorescence at different wavelengths or preferably suitable pairs of fluorescence donors/reporters and fluorescence quenchers/acceptors. The person of skill in the art is able to choose the most suitable detectable molecules for the respective application.

Another preferred embodiment of the invention is a method for the identification of a microorganism as originating from human and/or ruminant faeces in a sample, comprising a real time PCR using at least two oligonucleotides according to the invention, wherein one is used as a primer and the other is used as a probe.

It was found that specific sequences in the 16S rRNA gene of members of the group *Bacteroidetes* were dominant in human faeces and thus constituted ideal human markers for use with quantitative real-time PCR.

One of the key advances of the present invention is the use of a minor groove binder probe for a quantitative PCR assay, preferably in a microbial source tracking (MST) application. Preferably, the probes are very short with a length of usually 14 to 20 nucleotides while maintaining a high annealing temperature in the range of 65 to 70°C which is necessary for efficient 5'-nuclease quantitative PCR (Afonina et al., 1997; Kutyavin et al., 2000).

This is of special interest for assays and methods targeting genetic markers, because the conserved target sequences are often very short and not suitable for a 5'-nuclease PCR, where a standard TaqMan® probe has a length of usually between 25 and 35 nucleotides. The reduced length of a probe according to the present invention also increases the annealing temperature differential between matched and mismatched probes thereby improving specificity and ameliorates the signal to noise ratio of the fluorescence detection (Kutyavin et al., 2000; Marras et al., 2006).

The same advantages also apply to locked nucleic acid (LNA) and peptide nucleic acid (PNA). The TaqMan MGB probes can alternatively be used in LNA format, which allows for replacing the costly TaqMan MGB probes with the less expensive LNA probes (see Letertre et al. 2003). It is furthermore applicable to use PNA probes instead of the MGB probes. However, at the moment PNA are an expensive alternative due to their costly synthesis (see e.g. Kuhn et al. 2002).

It is especially preferred to use a real-time PCR standard (i.e. a quantification standard) with a defined amount of marker equivalents in every measurement run. This allows a reliable and above all reproducible quantification of marker equivalents (Mackay et al., 2002). The use of the oligonucleotides, kit and the method according to the present invention allows for detection of marker copies in the range of the theoretical limit of detection and therefore are perfectly able to meet the set requirement for the detection of faecal contamination in ground and spring water (especially with respect to human faecal pollution) and/or karstic spring water (especially with respect to ruminant faecal pollution).

The determination of the practical detection limit (as described below in Example 1) showed that despite the intermediate steps of filtration and DNA extraction the present invention made it possible to detect human faecal material in the extremely low range of from 68 to 909 picogram in any filtrated volume of water.

For ruminant faecal pollution, the determination of the practical detection limit (as described below in Example 2) showed that despite the intermediate steps of filtration and DNA extraction the present invention made it possible to detect faecal material in the unprecedented range of a few nanograms of faeces per filtrated volume of water. In comparison, Bernhard and Field (2000) determined detection limits in the range from 2.8 × 10⁻⁵ to 2.8 × 10⁻⁷ g faeces per litre in the PCR assay for their ruminant *Bacteroides* markers. In another publication (Savill et al., 2001), the detection limit of a quantitative real-time PCR assay for the detection of *Rhodococcus coprophilus* was 1 CFU per reaction. Since this bacterium is present at levels between 5.5 × 10³ and 3.6 × 10⁶ CFU per gram of herbivore faeces this method exhibits a sensitivity even lower than the mentioned conventional PCR assay for detection of *Bacteroides* marker (Bernhard and Field, 2000).

The high sensitivity of the presented method is also demonstrated by comparing it with cultivation based faecal indicators.

As an example in the context of human faecal pollution: the median value for marker equivalent concentrations in faeces were 4 × 10³ times higher than the median value for cultivable E. *coli* concentrations. In waste water and cesspit samples this median ratio was even higher with a value of 1 × 10⁶ (see Examples). An additional interesting observation is the much lower variablity of the human marker equivalent concentrations (approximately one order of magnitude) as compared to the variability of the *E. coli* counts (up to three orders of magnitude).

As an example in the context of ruminant faecal pollution: the concentration of cultivable *E. coli* in faeces from cattle, deer and chamois ranges from 4.0 × 10⁶ to 7.7 × 10⁷ CFU per g with an average of 2 × 10⁷ CFU per g (Famleitner et al., 2005) as compared to the average of 4.1 × 10⁹ ruminant marker equivalents per g as measured in the present invention (see Examples). This results in a 100 times higher sensitivity using the method of the present invention.

Furthermore, the only quantitative method available for the detection of human specific faecal DNA markers (Seurinck et al., 2005) exhibits a quantitative detection limit of 4.7 × 10⁵ markers per litre of freshwater. In contrast the method according to the invention reaches reliable quantitative results down to 6 × 10² human marker copies in any filtration volume, resulting in an almost 100 times higher sensitivity. The previous assay uses SYBR Green I for qPCR detection. This can cause various problems due to formation of false positives by detection of primer dimers (McCartney et al., 2003) to preferential binding of SYBR Green I to specific sequences (Giglio et al., 2003). In addition, this method uses only one specific primer and a reverse primer not specific for the marker sequence.

The fact that the human specific marker was detected in all waste water samples originating from all over Eastern Austria demonstrates the applicability of the assay for the source specific detection of faecal contamination. No animal faecal samples from the environment gave positive results. One result showing cross-species amplification of the marker sequence was obtained with a sample of cat faeces from a domestic cat kept in a city flat. This can be explained by the cats close relationship with its human keepers and their similar diet. However, this single cross-species amplification is not relevant for actual waste water pollution due to the fact that cats, especially domestic cats, do not form a relevant source of faecal pollution.

The prevalence of the detected (ruminant) marker among ruminants was 100% demonstrated on samples from cattle, deer and chamois, the dominant ruminant faecal sources in the area where most of the samples were taken (n = 37), as well as on numerous samples from ruminant animals collected in other areas of eastern Austria (n = 20). The assumption of a cosmopolitan occurrence of the ruminant marker is supported by studies from the United States (WO 02/27014, Bernhard and Field, 2000; Fogarty and Voytek, 2005). This also constitutes strong evidence that the strain carrying this marker is specifically adapted to ruminants as hosts (Gordon, 2001).

The specificity and high sensitivity of the oligonucleotides, kits and the methods, respectively, according to the present invention are highly useful in source tracking studies and investigations, especially in ground and spring water sources.

This is supported by the detection of the human marker in several natural aquatic systems in a karstic catchment region in the tests that were carried out by the inventors of the present invention. Strong differences in occurrence of the human marker in water samples were obvious and in accordance with the expected different levels of human faecal influence. This data also demonstrate the wide dynamic range of detection offered by the present invention. This is furthermore supported by the fact that the ruminant marker occurred in various aquatic environments throughout the study area. Strong differences in occurrence were obvious and in accordance with the expected different levels of ruminant faecal influence. The LKAS2 spring site (see below) showed low ruminant marker levels in early summer when faecal indicators were not detectable, while the levels during a strong summer flood event were very high corresponding to high faecal indicator counts in the same sample (1.6 × 10³ *E. coli* per litre, unpublished data). This demonstrates the wide range of detection possible with this assay.

As far as is known, no methods with comparable performance for the specific detection of human and/or ruminant faecal contamination have been available up to now. In addition, the methods according to the present invention are relatively fast and simple. They can be performed within half a day in any molecular biological lab with qPCR equipment. In contrast most established MST methods are either extremely laborious like library based typing methods (Simpson et al., 2002) or are not sensitive enough for spring water sources (Seurinck et al., 2005, Savill et al. 2001).

The present invention enables specific allocation of faecal pollution in a water source. This allows target-oriented measures in the catchment area, e.g. the reduction of livestock numbers in sensitive locations or the installation of treatment facilities or repair of septic systems or sewers, and thus facilitates watershed management. It might help waterworks to provide save drinking water by meeting the requirement for best source water quality set down in regulations (BMGF, 2001; Kralik, 2001).

The methods according to the present invention can also provide insights when used in quantitative microbial risk assessment (QMRA) studies as part of water safety plans recommended by the WHO (2004). In this field they allow the affirmation or exemption of human faecal input and respectively indicate or rule out the presence of human specific pathogens and/or they allow the relative estimation of ruminant faecal input as compared to other sources and indicate the potential presence of organisms causing zoonoses in humans (Sinton et al., 1998). They also allow the coverage of the largest portion of potential faecal sources in many regions (when detecting human and ruminant faecal pollution or when discriminating between them).

For the alpine karstic system preliminary experiments testing the stability of the marker in spring water at ambient temperatures of spring water were performed and no strong reduction of detectable marker levels were found during an incubation period of two months. This result is in accordance with earlier findings for human specific *Bacteroidetes* markers (Seurinck et al., 2005). However, in aquatic systems with higher temperature and trophic status an increased decay of the detectable marker may be expected.

The following example illustrate the present invention without, however, limiting the same thereto.

### BRIEF DESCRIPTION OF THE DRAWINGS

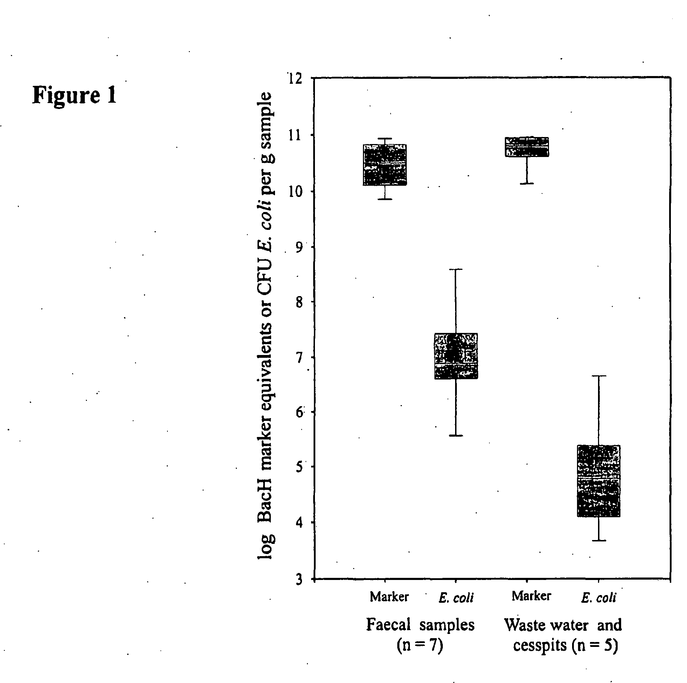

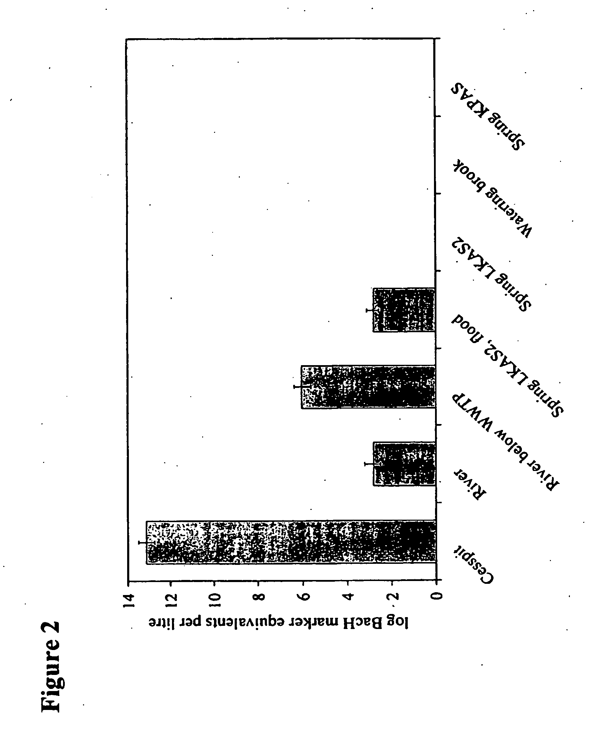

**Figure 1****:** *Detection and quantification of human faecal pollution in water.*
   The marker equivalent concentrations and E. *coli* numbers found in human faeces and in waste water and cesspit samples are illustrated. The results are given as log 10 of marker equivalents or CFU *E. coli* per g wet faeces or waste water, respectively. Whiskers indicate the range of concentrations, the boundaries of the box indicate the 25th percentile (lower limit) and the 75th percentile of measurements while the line within the box indicates the median value and n the number of samples measured per category
**Figure 2****:** *Detection and quantification of human faecal pollution in different samples taken throughout the study area.*
   The levels of human marker equivalent concentration measured in samples taken throughout the study area are illustrated. The results are given as average of log 10 marker equivalents per litre of filtered sample with ranges indicated by the whiskers. The filtration volumes for the samples are given in litres. In case of the cesspit, filtrations were performed from dilutions of the sample. Three independent filtrations and measurements were performed from each sample.

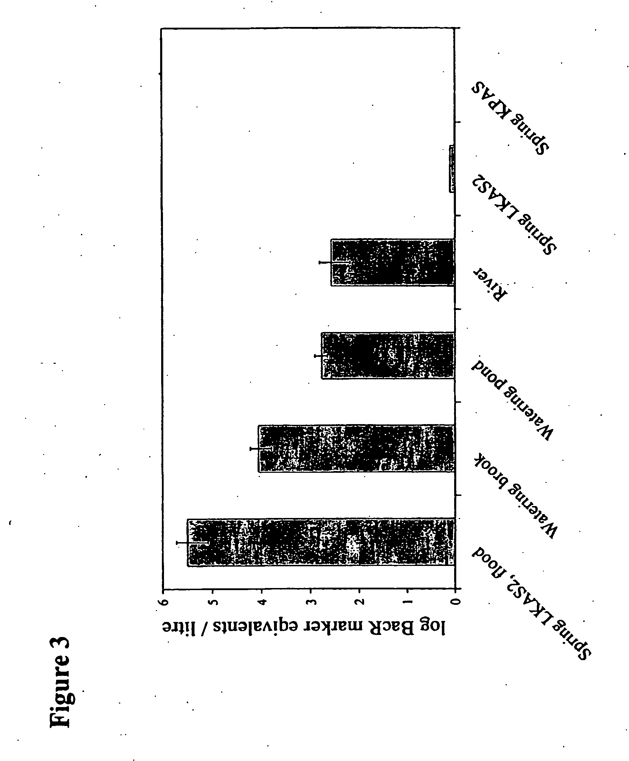

**Figure 3****:** *Detection and quantification of ruminant faecal pollution in different samples taken throughout the study area.*
   The levels of marker equivalent concentration measured in samples taken throughout the test area are illustrated. The results are given as average of log marker equivalents per litre of filtered sample with ranges as well as the filtration volume for the samples in litres. In sample Spring LKAS2, early summer marker equivalents were detectable close to the detection limit in the investigated volume, but not reliably quantifiable.

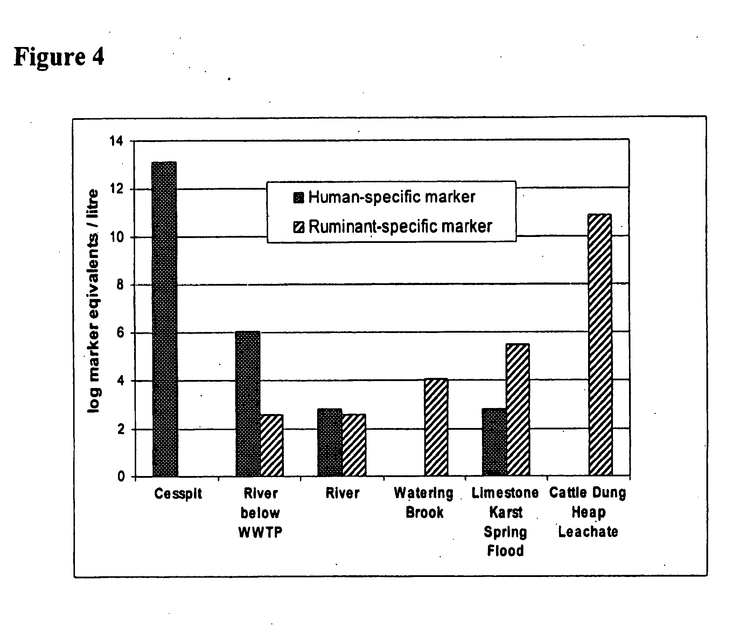

**Figure 4****:** *Detection and discrimination of human and ruminant faecal pollution in water.*
   The levels of human and ruminant marker equivalent concentration measured in samples taken throughout the test area are illustrated. Values are logarithms of marker equivalents for human-specific BacH marker (dark columns) and ruminant-specific BacR marker (striped columns).

### Example 1: Detection and quantification of human faecal pollution in water

### 1. Primer and probe design

The 16S rRNA gene sequence alignment generated from published *Bacteroidetes* sequences led to the development of the primers: forward primer BacH_f [SEQ ID NO: 4] and reverse primer BacH_r [containing SEQ ID NO: 5], see Table 1 and Section 6, Experimental procedures. These primers were tested in a conventional PCR assay on a small setup of DNA extracts from selected faecal samples collected throughout Eastern Austria in order to confirm the preliminary specificity of the primers alone (data not shown) and to obtain additional sequence information for qPCR probe design.

The 33 subcloned sequences belonged to two sequence types (Table 1). Approximately one third of the inserts showed type C while approximately two thirds showed type T sequence. Based on this information the TaqMan® MGB probes BacH_pC [SEQ ID NO: 1] and BacH_pT [SEQ ID NO: 2] were designed. For evaluation purposes the probes BacH_pC and BacH_pT were tested separately with their respective serial qPCR standard dilution series. The PCR efficiency was equally high for both independent assays (between 97 and 100 %). For the final assay both probes were combined in one reaction and tested by measuring both standards seperately and in mixtures. The probes showed no reduction in their capacity to quantify their the sum of the respective sequence types down to their detection limits in any mixing ratio.

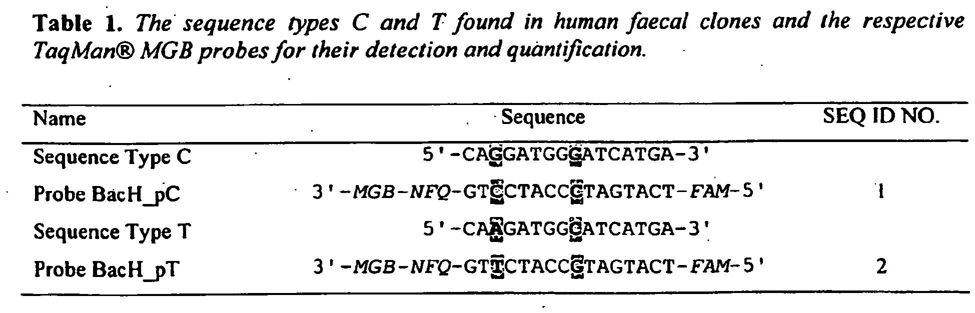

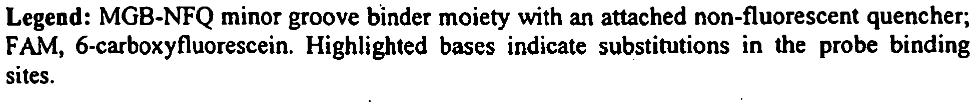

### 2. Characteristics of the PCR assay

Absolute quantification of the marker equivalents of the human-specific marker in the qPCR assay was achieved by analysing a dilution series of the plasmid standard with a known amount of marker equivalents in every measurement run. The marker equivalents of any unknown sample could be determined from the respective standard regression curves. The PCR efficiencies derived from the standard regression curves were between 97% and 100% for all runs (R² > 0.99). The lower qualitative detection limit was in the range of a few copies of the marker per reaction volume demonstrated by the fact that the standard containing 7 marker copies per reaction volume was detectable in all runs.

Reproducible and linear quantification was possible in the range from 30 to 10⁷ marker copies per reaction volume. A check for the possible presence of PCR inhibitors was made by analysing DNA from water and faecal samples in several 10-fold dilution steps. No inhibitory effects could be observed in any water samples allowing the use of undiluted DNA extracts. For faecal samples 10 representative faecal DNA extracts were tested in four serial 10-fold dilutions. Inhibition was apparent in the undiluted faecal DNA extracts. In order to rule out any inhibition 100-fold diluted faecal DNA extracts were used in the test of specificity.

### 3. Specificity of the PCR assay

The specificity of the assay was tested on DNA extracted from human faecal samples, cesspit and waste water samples as well as animal faecal samples (Table 2). Most of the cesspit samples and a majority of the animal samples originated from an alpine catchment area in the Northern calcareous alps with a size of approximately 100 km², while all human single samples and several waste water and animal samples were collected in the much larger region of Eastern Austria (around 25000 km²).

The marker sequence was detected in 100% of the municipal waste water and cesspit samples from mountain huts (n = 20; Table 2). It was present in 20 out of 21 analysed faecal samples from human individuals, wherein the one negative sample is considered to be an outlier. However, since the assay was developed for detecting/quantifying human faecal pollution in environmental samples, such as waste water samples, which are preferably pooled samples and no individual samples, this result does not affect the suitability of this assay. The marker was not detectable in any animal faecal sample tested (n = 302) with the exception of one sample from a domestic cat kept in a city flat. This can be explained by the cats close relationship with its human keepers and their similar diet (Table 2). Agarose gel electrophoresis was used on all reaction products in this experiment to rule out the presence false-negative results.

**Table 2. Specificity of the PCR assay tested on human and animal faecal samples as well as human pooled samples from cesspits and waste water.**

| Source | Scientific name | Sample type ^{a} | Number of samples ^{b} | BacH positive | BacH negative |
|---|---|---|---|---|---|
| Cesspits and wastewater ^{c} | *Homo sapiens* | wastewater | 20 | 20 | - |
| Human individual samples ^{d} | *Homo sapiens* | single | 21 | 20 | 1 |
| | | | | | |
| Cattle | *Bos taurus* | pooled | 6 (60) | - | 6 |
| Deer | *Cervus elaphus* | pooled | 4 (40) | - | 4 |
| Chamois | *Rupicapra* | pooled | 4 (40) | - | 4 |
| Roe deer | *Capreolus* | pooled | 1 (10) | - | 1 |
| Sheep | *Ovis aries* | pooled | 3 (30) | - | 3 |
| Goat | *Capra hircus* | pooled | 3 (30) | - | 3 |
| Horse | *Equus caballus* | pooled | 3 (30) | | 3 |
| Fox | *Vulpes vulpes* | single | 2 | - | 2 |
| Dog | *Canis familiaris* | single | 3 | - | 3 |
| Cat | *Felis catus* | single | 3 | 1 | 2 |
| Pig | *Sus scrofa* | pooled | 2 (20) | - | 2 |
| Chicken | *Gallus gallus* | pooled | 1 (10) | - | 1 |
| Turkey | *Meleagris* | pooled | 1 (10) | - | 1 |
| Swan | *Cygnus* | single | 1 | - | 1 |
| Duck | *Anas* | pooled | 1 (10) | - | 1 |
| Black grouse | *Tetrao tetrix* | single | 3 | - | 3 |

| | | | | | |
|---|---|---|---|---|---|
| **Legend:** ^{a} single: single faecal sample; pooled: sample pooled from 10 single faecal samples of the respective source or waste water sample; ^{b} numbers in brackets indicate the original number of samples before pooling; ^{c} cesspits from mountain huts in the study area with 10 to 100 inhabitants, municipal waste water from settlements with 300 to 2 million inhabitants from all over Eastern Austria; ^{d} single faecal samples from persons living all over Eastern Austria. | | | | | |

### 4. Limit of detection in applied use

It was not possible to relate the marker equivalents of the human marker directly to a cell count of the *Bacteroidetes* cells carrying this marker because this strain has not been isolated yet. The results were therefore expressed as marker equivalents detected in the qPCR assay, and the number of those equivalents present in human faeces, waste water and cesspit samples suspended in water was assessed as the environmental matrix of interest.

One gram of faeces from seven human single samples and five waste water or cesspit samples was suspended and diluted in 0.2 µm pre-filtered spring water in 100-fold dilution steps. After filtration and DNA extraction the marker equivalent concentration was determined, signifying the actual equivalents of marker still detectable after the losses incurred by the preceding steps. In addition, *E. coli* enumeration was performed from the same samples.

The marker equivalent concentrations detected in human faecal samples ranged from 6.6 × 10⁹ - 8.7 × 10¹⁰ marker equivalents per g wet faeces, for waste water samples the range was 1.4 × 10¹⁰ - 9.1 × 10¹⁰ marker equivalents per g waste water and cesspit samples (Figure 1).. These values were determined from the most diluted faecal suspensions showing positive and quantifiable qPCR results. The marker was not detected in any higher dilutions nor in the negative controls (the filtered spring water used for dilution). The faecal samples contained cultivable *E. coli* in concentrations between 2.0 × 10⁵ and 7.5 × 10⁸ CFU per g wet faeces, while cesspit and waste water samples contained between 4.7 × 10³ to 4.5 × 10⁶ CFU E. *coli* per gram sample (Figure 1).

From the results of the marker equivalent concentrations the detectable concentrations of recent human faecal pollution in water were extrapolated, assuming a sample with 6 marker equivalents per reaction (for a 95% detection frequency) using the corresponding standard regression curve. The average detection limit for human faecal input was 3.2 × 10⁻¹⁰ g of wet faeces and 1.6 × 10⁻¹⁰ g waste water per analysed filter.

### 5. Occurrence of the marker throughout the test area

To investigate the quantitative occurrence of the human marker in natural aquatic systems water samples were taken from selected sites in and around a karstic spring catchment area. Sampling locations were chosen according to a presumptive faecal pollution gradient. The sampling sites ranged from well protected springs to waste water. The marker could be found at concentrations ranging from not detectable in 4.5 litres to 10¹³ marker equivalents per litre (Figure 2).

It was not detectable in the well protected spring water of Spring KPAS, in the sample from Spring LKAS2 taken during winter and in a watering brook inside a remote fenced game feeding compound. The levels in a Spring LKAS2 sample taken during a strong summer flood event and in river water were in the range of 10² - 10³ marker copies per litre. Higher marker concentrations were found in a river sample taken right below a waste water treatment plant of a small village in the study area. The highest marker concentrations were detected in a waste water sample taken from the cesspit of a highly frequented mountain hut (Figure 2).

### 6. Experimental procedures

### a) Faecal sampling and DNA extraction

Faecal, cesspit and wastewater samples were collected in a large alpine catchment area in the Northern Calcareous Alps covering an area of approximately 100 km³. Additional samples including most of the human individual samples for assessment of the potential broader applicability originate from the larger area of Eastern Austria. The total number of samples was 361. For pooled faecal samples 10 individual samples were combined in a sampling tube and homogenised. Samples were taken in sterile faecal sampling tubes and stored at -20 °C. DNA was extracted from 100 mg of each faecal sample using the Ultra Clean Soil DNA kit (MoBio Laboratories, Inc. Carlsbad, USA) in combination with bead-beating (FastPrep^{™} FP120, Bio-101, Vista, USA) following the manufacturer's recommendations. Cell lysis was performed at a machine speed setting of 6 for 30 seconds. DNA was stored at -20 °C.

### b) Water sampling and.DNA extraction

Water samples were taken in clean and autoclaved Nalgene (Nalge Europe Ltd., Hereford, UK) sampling bottles, stored in dark cooling boxes at 4 °C for transportation and processed within 6 h after collection. For molecular biological analysis a given volume of the water sample was filtered through polycarbonate membrane filters (Isopore™, 45 mm diameter, 0.2 µm pore size, Millipore Corp. Bedford, USA). Three independent filtrations were done for each sample. Immediately after filtration, filters were frozen and stored at -80°C until nucleic acid extraction. Nucleic acid extraction was performed as described by Griffiths et al. (2000), using isopropanol instead of the polyethylene glycol for DNA precipitation. Recovered DNA was redissolved in 50 µl of sterile bi-distilled water and stored at -80 °C until further analysis. All analysed DNA extracts of faecal and aquatic origin contained amplifiable bacterial DNA as checked by applying a universal bacterial PCR assay (Teske et al., 1996).

### c) Primer design

Primers were designed by aligning the following published 16S rRNA gene sequences using the Vector NTI software package (InforMax Inc., Frederick, USA): AF233408, AF233409, AF233410, AF233411, AF233412, AF233413 (Bernhard and Field, 2000a) Primers were designed from the derived consensus sequence with the Primer Express^{®} software (Applied Biosystems, Foster City, USA). The primers used in the assays described herein were designated BacH_f (5'- CTTGGCCAGCCTTCTGAAAG -3') [SEQ ID NO: 4] and BacH_r (5'-CCCCATCGTCTACCGAAAATAC-3') [containing SEQ ID NO: 5].

### d) Conventional PCR

The designed primers were used to amplify a 93 bp fragment from 10⁻² dilutions of DNA extracts from cesspits, waste water and faecal samples of human individuals (total n = 18). PCR was performed on the iCycler iQ Real-Time Detection System (Biorad, Hercules, USA). Reaction mixtures (25 µl total volume) contained 2.5 µl of template DNA dilution, 200 nM BacH_f, 200 nM BacH_r, 10 µg bovine serum albumin (Boehringer Mannheim, Mannheim, Germany), 12.5 µl of iQ Supermix (Biorad) and PCR grade water (Roche Diagnostics, Vienna, Austria) to a final volume of 25 µl. The PCR program was the following: 95 °C for 3 min, 30 cycles of 95 °C for 15 s, 61 °C for 15 s and 72 °C for 45 s followed by a final extension at 72 °C for 3 min. All PCR products were analysed by agarose gel electrophoresis to check for correct amplicon size.

### e) Cloning and sequencing

A total of 14 conventional PCR assays with the newly developed primers were performed from DNA of three human single samples, one pooled sample composed of five human single samples, one pooled sample containing samples from three cesspits, three samples from individual cesspits and six samples of municipal waste water (populations of 20, 600, 1500, 7000, 23000 and 2 million, respectively). PCR amplicons were cloned into a pGEM-T Vector (Promega, Madison, USA). After transformation of *Escherichia coli* JM 109 with the plasmid and purification of the plasmid DNA with the Quantum Prep Plasmid Miniprep Kit (Biorad) at least two clones per sample were commercially sequenced (MWG-Biotech, Ebersberg, Germany). Sequences are available at GenBank under accession numbers DQ384022 - DQ384054.

### f) Probe design

The sequences gained from the faecal DNA were included in the sequence alignment. It was decided to design two separate but identically labelled TaqMan® MGB probes for the detection and quantification of both dominant sequence types. The probes named BacH_pC (FAM-5'-TCATGATCCCATCCTG-3'-NFQ-MGB) [SEQ ID NO: 1] and BacH_pT (FAM-5'- TCATGATGCCATCTTG -3'-NFQ-MGB) [SEQ ID NO: 2] (Table 1) were designed using the Primer Express® software, with FAM being 6-carboxyfluorescein and NFQ-MGB a non-fluorescent quencher with an attached minor groove binder moiety, and synthesized at Applied Biosystems (Warrington, UK). The primers and probes were analysed for specificity *in-silico* by basic local alignment search tool analysis using the NCBI Blast feature (blastn; http://www.ncbi.nlm.nih.gov/blast) and the amplicon was analysed for intra- and intermolecular secundary structures using the mfold software (http://www.bioinfo.rpi.edu/applications/mfold/dna/form1.cgi).

### g) Real-time standard

Two of the specific clones (GenBank accession numbers DQ384034 and DQ384048 for BacH_pC and for BacH_pT reference standards, respectively) were used for the generation of plasmid standards for qPCR. After re-transformation of competent *E. coli* with the plasmids and purification of the plasmid DNA with the Plasmid Midi Kit (Qiagen, Hilden, Germany), the concentration of the plasmid standard solutions were measured on an UV spectrophotometer (Hitachi Ltd, Finchamstead, UK) and the standards were diluted in 10-fold steps in a 5 ng/µl poly d(I-C) solution (Roche Diagnostics) as unspecific DNA background.

### h) Real-time PCR procedure

QPCR was monitored on an iCycler iQ Real-Time Detection System. Assay optimisation included a Mg²⁺ concentration gradient between 3 mM and 5 mM, an annealing temperature gradient between 55 °C and 68 °C and a primer matrix experiment with primer concentrations ranging from 100 nM to 500 nM. Optimised reaction mixture composition contained 2.5 µl of template DNA dilution, 200 nM BacH_f, 200 nM BacH_r, 100 nM BacH_pC, 100 nM BacH_pT, 10 µg bovine serum albumin (Boehringer Mannheim), 12.5 µl of iQ Supermix (Biorad) and PCR grade water (Roche Diagnostics) to a final volume of 25 µl. The PCR program was the following: 95 °C for 3 min, 50 cycles of 95 °C for 15 s, 61°C for 15 s and 72 °C for 45 s.

Real-time data were collected during the elongation step at 72 °C. All reactions were performed in triplicates and in at least two tenfold dilution steps of the template DNA. A total of six tenfold serial dilutions of the BacH_pT standard (7 to 7 × 10⁵ gene copies) were run in triplicate on every wellplate as well as a no-template control and a no-amplification control (containing plasmid standard and 0.01% sodium dodecyl sulphate). For statistical considerations the following formula for an approximative cumulative Poisson distribution was applied according to Lorenz (1992): S = Xₘ ± √Xₘ where S is the 95% confidence interval and Xₘ the arithmetic average.

### i) Limit of detection in applied use

Raw water from an alpine karst spring was filtered through a 0.22 µm Steritop™ membrane filter (Millipore Corp., Billerica, USA). Seven single faecal samples from humans living in different areas of Eastern Austria and five waste water samples from cesspits and sewers (number of inhabitants ranging from 10 to 100) were analysed in this experiment. One gram of wet faeces or waste water from each sample was suspended in 50 ml of filtered spring water and 100-fold dilution steps were prepared using the same water. One millilitre from each dilution step was filtered through 0.2 µm polycarbonate membrane filters (Millipore), DNA was extracted and qPCR was performed following the procedures described above. Additionally 100 and 500 ml of the spring water used for the suspensions were filtered and analysed as a negative control. *E. coli* concentrations as CFU per gram sample were determined from suspensions of the same samples as described in (Farnleitner et al., 2005).

### i) Occurrence of marker throughout the test area

Samples were taken from the following water sources in the study area: Spring KPAS, a very well protected spring site with a karstic catchment and a porous aquifer structure; Spring LKAS2, a relatively vulnerable karstic spring site with a mean water residence time estimated between 0.8 and 1.5 years and a very quick discharge response after precipitation; a small river in the study area was sampled upstream (River) and downstream of the effluent pipe of a waste water treatment plant treating waste water from a small village (population of 600; River below WWTP); a brook running through a remote game-feeding compound (Watering brook); the pit latrine cesspit of a mountain hut highly frequented by mountaineers (10-100 per day) during the summer months (Cesspit).

All sites were sampled once. Spring KPAS was sampled in November 2005 and Spring LKAS2 in winter 2004 during base flow conditions. Spring LKAS2 was additionally sampled in summer 2005 during a flood event after heavy rainfall in the catchment area. The river and the watering brook were sampled in December 2005 after the start of the game feeding season. The cesspit was sampled in summer 2005. The filtration volumes lay between 0.3 and 4.5 litres, depending on turbidity. The cesspit sample was diluted and filtered as described in the detection limit section.

### k) Persistence of the marker in spring water

For the investigation of the persistence of the human specific marker in spring water at ambient conditions, a cesspit sample from a mountain hut was suspended in fresh spring water and diluted in a total volume of 25 litres to a final concentration of 1.6 × 10⁻⁵ g waste water per litre. Suspensions were kept at 4 °C and 1 litre samples were taken sterilely after 4, 11, 19, 27, 33 and 67 days. These samples were filtered, DNA was extracted and marker concentration was detected as described above.

### Example 2: Detection and quantification of ruminant faecal pollution in water

### 1. Primer and probe design

The 16S rRNA sequence alignment generated from published sequences led to the development of the primers: forward primer BacR_f [SEQ ID NO: 6] and reverse primer BacR_r [containing SEQ ID NO: 7], see Table 3 and Section 6, Experimental procedures. These primers were tested in a conventional PCR assay on a small setup of DNA extracts from selected faecal samples collected throughout the test area in order to assess preliminary specificity of the primers alone (date not shown) and to obtain additional sequence information for real-time PCR probe design.

The sequences retrieved by cloning showed very high similarity even between different ruminant sources. The 15 sequenced clones had an average length of 82 bp and showed sequence identities above 74%. This sequence information from ruminant faecal samples allowed the design of the TaqMan® MGB-probe BacR_p [SEQ ID NO: 3] (see Table 3). Subsequent real-time PCR optimisation proved that a Mg²⁺ concentration of 5 mM and a primer ratio of at least 1:5 between primers BacR_f and BacR_r was necessary for efficient amplification.

**Table 3. Primers and probe for the detection of ruminant faecal pollution according to the present invention.**

| Name | Function | SEQ ID NO: | Sequence | Length | Annealing temp (°C) |
|---|---|---|---|---|---|
| BacR_f | Forward Primer | 6 | 5' -GCGTATCCAACCTTCCCG-3' | 18 bp | 58 |
| BacR_r | Reverse Primer TaqMan | 3 | 5'-CATCCCCATCCGTTACCG-3' | 18 bp | 58 |
| BacR_p | MGB probe | | FAM-5' -CTTCCGAAAGGGAGATT-3' -NFQ-MGB | 17 bp | 68 |

| | | | | | |
|---|---|---|---|---|---|
| **Legend**: FAM, 6-carboxyfluorescein; NFQ, non-fluorescent quencher; MGB minor groove binder moiety. | | | | | |

### 2. Characteristics of the PCR assay

Absolute quantification of the marker equivalents of the ruminant specific marker in the PCR assay was achieved by analysing a dilution series of the plasmid standard with a known amount of marker equivalents in every measurement run. The marker equivalents of any unknown sample could be determined from the respective standard regression curves. The PCR efficiencies derived from the standard regression curves were above > 98% for all runs with respective correlation coefficients higher than 0.99. The lower detection limit was in the range of a few copies of the marker per reaction volume demonstrated by the fact that the standard dilution containing 5 plasmid copies per reaction volume was detectable in all runs.

Assuming a Poisson distribution in the samples a 95% detection probability can be expected in PCR reactions containing an average of 6 or more copies of the marker. This limit is derived from a approximate cumulative Poisson distribution with a 95% confidence interval as described in the experimental procedures. Reproducible and linear quantification was possible in the range from 20 to 10⁷ marker copies per reaction volume. A check for the possible presence of PCR inhibitors was made by analysing DNA from water and faecal samples in several 10-fold dilution steps. For water samples no inhibitory effects could be observed in any of them. Therefore undiluted DNA extracts were used in analysis.

For faecal samples 10 representative faecal DNA extracts were tested in four 10-fold dilution steps. Inhibition was apparent in the undiluted DNA extract. In order to rule out any inhibition 100-fold diluted faecal DNA extracts were used in the test of specificity.

### 3. Specificity of the PCR assay

To assess the specificity of the assay it was applied on DNA extracted from faecal samples from ruminant and non-ruminant animals as well as human samples (Table 4). Among the group of ruminants, single samples were tested to measure the frequency of presence of the marker in ruminant faecal samples. For all other groups of sources, pooled samples, containing 10 individual samples, were preferred whenever available in order to prove the absence of the marker in a larger number of samples with a single reaction. The marker sequence was shown to be present in 100% of the ruminant sources (47 samples tested). No amplification was achieved from any non-ruminant source (101 non-ruminant animal samples, 30 samples from human individuals, 13 samples from cesspits of alpine huts and 2 samples of municipal waste water from a small village in the test area) (Table 4).

**Table 4. Specificity of the BacR real-time PCR assay tested on ruminant and non-ruminant animal faecal samples as well as human pooled faecal samples from cesspits and waste water.**

| Source | Scientific name | Sample type ^{a} | Number of samples ^{b} | BacR positive | BacR negative |
|---|---|---|---|---|---|
| Cattle | *Bos taurus* | single | 14 | 14 | - |
| Deer | *Cervus elaphus* | single | 12 | 12 | - |
| Chamois | *Rupicapra rupicapra* | single | 12 | 12 | - |
| Roe deer | *Capreolus capreolus* | single | 6 | 6 | - |
| Sheep | *Ovis aries* | single | 6 | 6 | - |
| Goat | *Capra hircus* | single | 5 | 5 | - |
| Capricorn | *Capra ibex* | single | 2 | 2 | - |
| | | | | | |
| Cesspits and | *Homo sapiens* | wastewater | 15 | - | 15 |
| Human | *Homo sapiens* | pooled | 3 (30) | - | 3 |
| Horse | *Equus caballus* | pooled | 3 (30) | - | 3 |
| Pig | *Sus scrofa domestica* | pooled | 3 (30) | - | 3 |
| Fox | *Vulpes vulpes* | single | 2 | - | 2 |
| Cat | *Felis catus* | single | 3 | - | 3 |
| Dog | *Canis familiaris* | single | 3 | - | 3 |
| Chicken | *Gallus gallus* | pooled | 1 (10) | - | 1 |
| Turkey | *Meleagris gallopavo* | pooled | 1 (10) | - | 1 |
| Swan | *Cygnus sp.* | single | 1 | - | 1 |
| Duck | *Anas platyrhynchos* | pooled | 1 (10) | - | 1 |
| Black grouse | *Tetrao tetrix* | single | 2 | - | 2 |

| | | | | | |
|---|---|---|---|---|---|
| **Legend**: ^{a} single: single faecal sample; pooled: sample pooled from 10 individual faecal samples of the respective source or waste water sample; ^{b} numbers in brackets indicate the original sample number before pooling; about 50% of the samples used in this experiment were collected in an alpine karstic catchment area, the rest originated from all over Eastern Austria. | | | | | |

### 4. Limit of detection in applied use

It was not possible to relate the marker equivalents of the ruminant marker directly to a cell count of the *Bacteroidetes* cells carrying this marker, because this strain has not been isolated yet. The results were therefore expressed as marker equivalents detected in the PCR assay, and the number of those equivalents present in ruminant faeces suspended in water was assessed as the environmental matrix of interest.

A known amount of faecal material from 9 pooled samples from the relevant ruminant animals cattle, deer and chamois was suspended and diluted in 0,2 pre-filtered spring water. After filtration and DNA extraction the marker equivalent concentration was determined, signifying the actual equivalents of marker still detectable after the losses incurred by the preceding steps.

Detection was possible down to a filtered volume containing 2 × 10⁻⁸ g faeces showing relatively similar marker equivalent numbers in all 9 pooled samples. In dilution steps with lower concentrations the marker was not detectable in any sample. The marker was present in ruminant faeces at an average concentration of 4.1 × 10⁹ marker equivalents per g wet faeces. The values showed little variation, which is rather a consequence of the pooling process than attributable to population dynamics (Table 5).

**Table 5. Marker equivalent concentrations in suspended faeces and detection limits in suspensions.**

| Source | Number of samples | Marker equivalents in suspended faeces (10⁹ copies /g faeces) | | Detectable concentration^{a} (10⁻⁹ g faeces/filter) | |
|---|---|---|---|---|---|
| | | Average | Range | Average | Range |
| Cattle | 3 | 3.7 | 3.0 - 4,3 | 1.7 | 1.4 - 2.0 |
| Deer | 3 | 4.5 | 3.8 - 5.7 | 1.4 | 1.1 - 1.6 |
| Chamois | 3 | 4.2 | 1.6 - 6.7 | 2.0 | 0.9 - 3.9 |

| | | | | | |
|---|---|---|---|---|---|
| **Legend**: ^{a} Detection limits were calculated as the minimum amount of faeces detectable in a filtration volume assuming a 95% detection probability based on a Poisson distribution, i.e. 6 marker equivalents per PCR reaction volume. They are the reciprocal value of the marker equivalents per g faeces multiplied by 6 for the minimum average marker equivalent number reliably detectable. | | | | | |

From these results the detectable concentrations of ruminant faecal pollution in water assuming a sample with 6 marker equivalents per reaction were extrapolated using the corresponding standard regression curve. The results showed that it is possible to detect faecal input in the range of 0.9 - 3.9 × 10⁻⁹ g wet faeces per analysed filter with an average of 1.7 × 10⁻⁹ g (Table 5).

### 5. Occurrence of the marker throughout the test area

To investigate the quantitative occurrence of the ruminant marker in the selected test area water samples were taken from selected aquatic habitats covering a presumptive faecal pollution gradient. The sampling sites ranged from well protected springs to heavily influenced surface waters. The marker could be found at concentrations ranging from not detectable in 4.5 litres to 10⁶ marker equivalents per litre (Figure 3).

It was not detectable in the well protected spring water of Spring KPAS. It was present in levels close to the detection limit in 4.5 litres of water from Spring LKAS2 during slightly elevated flow conditions in early summer. The levels in river water and in a watering pool inside a game-feeding compound during times of normal discharge without recent precipitation were comparable and were in the range of 10²- 10³ marker copies per litre, while the watering brook site showed slightly higher concentrations. The highest marker concentrations were found in the Spring LKAS2 taken during an extraordinarily strong summer flood event (3 × 14⁵ marker equivalents per litre) (Figure 3 and Table 4).

**Table 4. Levels of ruminant marker equivalent concentration measured in samples taken throughout the study area (3 filtrations per sample).**

| Sampling site | Filtration volume (I) | Marker equivalents per litre Average | Marker equivalents per litre Range |
|---|---|---|---|
| Spring LKAS2, flood | 1.5 | 3.2 × 10⁵ | 1.2 - 5.3 × 10⁵ |
| Watering brook | 1.0 | 1.2 × 10⁴ | 0.6 - 1.7 × 10⁴ |
| Watering pond | 2.0 | 5.9 × 10² | 4.4 - 8.1 × 10² |
| River | 0.3 | 3.7 × 10² | 1.5 - 6.5 × 10² |
| Spring LKAS2 | 4.5 | detected ^{a} | detected ^{a} |
| Spring KPAS | 4.5 | ND ^{b} | ND ^{b} |

| | | | |
|---|---|---|---|
| ^{a} detectable close to detection limit in the investigated volume. ^{b} ND, not detectable. | | | |

### 6. Experimental procedures

### a) Faecal sampling and DNA extraction

Faecal samples were collected in a large alpine catchment area in the Northern Calcareous Alps covering an area of approximately 100 km³ (for description of test area see Farnleitner et al. 2005). Additional samples for assessment of the broader applicability originate from the larger area of eastern Austria. For pooled faecal samples 10 individual samples were combined in a sampling tube and mixed thoroughly. Samples were collected in sterile faecal sampling tubes and stored at -20°C. DNA was extracted from 100 mg of each faecal sample using the Ultra Clean Soil DNA kit (MoBio Laboratories, Inc. Carlsbad, CA) in combination with bead-beating (FastPrep™ FP120, Bio-101, Vista, CA) following the manufacturer's recommendations. Cell lysis was performed at a machine speed setting of 6 for 30 seconds. DNA was stored at -20°C.

### b) Water sampling and DNA extraction

Water samples were collected in clean and autoclaved Nalgene (Nalge Europe Ltd., Hereford, UK) sampling bottles, stored in dark cooling boxes at 4°C during transport and processed within 6 h after collection. For molecular biological analysis a given volume of spring water was filtered through polycarbonate membrane filters (Isopore™, 45 mm diameter, 0.2 µm pore size, Millipore Corp., Bedford, MA). Immediately after filtration, filters were frozen and stored at -80°C until nucleic acid extraction. Nucleic acid extraction was performed as described by Griffiths et al. (2000), with a DNA precipitation using isopropanol instead of the polyethylene glycol. Recovered DNA was redissolved in 50 µl of sterile bi-distilled water and stored at -80°C until further analysis. All DNA extracts of faecal and aquatic origin were checked for the presence of amplifiable bacterial DNA by applying a universal bacterial PCR assay (Teske et al., 1996).

### c) Primer design

Primers were designed by aligning the following published 16S rRNA gene sequences using the Vector NTI software package (InforMax Inc., Frederick, MD): AF233400, AF233402, AF233403, AF233404 (Bernhard and Field, 2000a); AF294903, AF294904, AF294905, AF294906, AF294908 and AF294909 (Bernhard and Field, 2000b). Primers were designed from the derived consensus sequence with the Primer Express® software (Applied Biosystems, Foster City, CA). The primers used in the assays described herein were designated BacR_f (5'-GCGTATCCAACCTTCCCG-3') [SEQ ID NO: 6] and BacR_r (5'-CATCCCCATCCGTTACCG-3') [containing SEQ ID NO: 7] (see Table 3).

### d) Conventional PCR

The designed primers were used to amplify a 118 bp fragment from 10⁻² dilutions of DNA extracts from ruminant as well as non-ruminant faecal samples. PCR was performed on an iCycler iQ Real-Time Detection System (Biorad, Hercules, CA). Reaction mixtures contained 2.5 µl of template DNA dilution, 200 nM BacR_f, 200 nM BacR_r, 10 µg bovine serum albumin (Boehringer Mannheim, Germany), 12.5 µl of iQ Supermix (Biorad) and PCR grade water (Roche Diagnostics, Vienna, Austria) to a final volume of 25 µl. The PCR program was the following: 95°C for 3 min, 30 cycles of 95°C for 15 s, 60°C for 15 s and 72°C for 45 s followed by a final extension at 72°C for 3 min. All PCR products were analysed by agarose gel electrophoresis to check for correct size of the amplicons.

### e) Cloning and sequencing

PCR was performed from DNA of two single and one pooled faecal sample each for cattle, deer and chamois from representatively selected locations in the catchment area. PCR amplicons were then cloned into a pGEM-T Vector (Promega, Madison, WI). After transformation of *Escherichia coli* JM 109 with the plasmid and purification of the plasmid DNA with the Quantum Prep Plasmid Miniprep Kit (Biorad) the cloned inserts were sequenced by MWG-Biotech (Ebersberg, Germany). Sequences are available at GenBank under accession numbers DQ364808 - DQ364822.

### f) Probe design

The sequences gained from faecal DNA were included in the sequence alignment. The TaqMan® MGB probe BacR_p (FAM-5'-CTTCCGAAAGGGAGATT-3'-NFQ-MGB) [SEQ ID NO: 3] was designed using the Primer Express® software, with FAM being 6-carboxyfluorescein and NFQ-MGB a non-fluorescent quencher with an attached minor groove binder moiety (Applied Biosystems, Warrington, UK). The primers and probe were analysed for specificity *in-silico* by basic local alignment search tool analysis using the NCBI Blast feature (http://www.ncbi.nlm.nih.gov/BLAST).

### e) Real-time standard

One of the specific clones (GenBank accession number DQ364808) was used for the generation of a plasmid standard for real-time detection. After re-transformation of competent *E. coli* with the plasmid and purification of the plasmid DNA with the Plasmid Midi Kit (Qiagen, Hilden, Germany), the concentration of the plasmid standard solution was measured photometrically on a UV spectrophotometer (Hitachi Ltd, Finchamstead, UK) and the standard was diluted in 10-fold steps in a 10 ng/µl poly d(I-C) solution as unspecific DNA background (Roche Diagnostics, Mannheim, Germany).

### h) Real-time PCR procedure

PCR was monitored on an iCycler iQ Real-Time Detection System. PCR assay optimisation included a Mg²⁺concentration gradient between 3 mM and 5 mM, an annealing temperature gradient between 55°C and 68°C and a primer matrix experiment with primer concentrations ranging from 50nM to 500nM. Optimised reaction mixture composition contained 2.5 µl of template DNA dilution, 100 nM BacR_f, 500 nM BacR_r, 100 nM BacR_p, 10 µg bovine serum albumin (Boehringer Mannheim), 2 mM additional MgCl₂ (Biorad), 12.5 µl of iQ Supermix (Biorad) and PCR grade water (Roche Diagnostics) to a final volume of 25 µl. The PCR program was the following: 95°C for 3 min, 50 cycles of 95°C for 15 s, 60°C for 15 s and 72°C for 45 s.

Real-time data were collected during the elongation step at 72°C. All reactions were performed in triplicates and in two dilution steps. A total of six 10-fold dilution steps of plasmid standard (5 to 5 × 10⁵ gene copies) were run in triplicate on every well plate as well as a no-template control (water instead of sample) and a no-amplification control (containing plasmid standard and 0.01% SDS). For statistical considerations the following formula for an approximate cumulative Poisson distribution was applied according to Lorenz (1992): S = Xₘ ± √Xₘ where S is the 95% confidence interval and Xₘ the average.

### i) Specificity of the assay

DNA extracted from faecal samples and waste water was diluted 100-fold with sterile bi-distilled water and the marker was detected with the PCR assay.

### j) Limit of detection in applied use

Raw water from an alpine karst spring was filtered through a 0.22 µm Steritop™ membrane filter (MILLIPORE Corp., Billerica, MA). Three pooled faecal samples (each consisting of 10 individual samples) from cattle, deer and chamois, each, were analysed in this experiment. Pooled samples were chosen in order to equalise expected population dynamics of *Bacteroidetes* populations. One gram of wet faeces of each sample was suspended in 50 ml of filtered spring water and 100fold dilution steps down to a concentration of 2×10⁻¹⁰ g wet faeces per ml were prepared using the same water. One millilitre from each dilution step was filtered through 0.2 µm polycarbonate membrane filters (Millipore), DNA was extracted and real-time PCR measurements were performed following the procedures described above. Additionally 100 and 500 ml of the filtered spring water were filtered and analysed as a negative control.

### k) Occurrence of marker throughout the test area

Samples were taken from the following water sources in the test area: Spring KPAS, a very well protected spring site with a karstic catchment and a porous aquifer structure; Spring LKAS2, a relatively vulnerable karstic spring site with a mean water residence time estimated between 0.8 and 1.5 years and a very quick discharge response after precipitation; a small river in the study area, influenced by several villages (River); a watering pond inside a fenced game-feeding compound (Watering pond); a brook running through a different remote game-feeding compound (Watering brook). All sites were sampled once. Spring KPAS was sampled in November 2005 and Spring LKAS2 in early summer 2005, during elevated flow conditions. Spring LKAS2 was additionally sampled in Summer 2005 during a flood event after heavy rainfall in the catchment's area. The river as well as both watering sites were sampled in December 2005 after the start of the game feeding season. Three separate filtrations and marker measurements were performed with each sample. The filtration volumes lay between 0.3 and 4.5 litres, depending on turbidity.

### 1) Persistence of the marker in spring water

For the investigation of the persistence of the ruminant specific marker in spring water at ambient conditions, pooled faecal samples from cattle and chamois were suspended in fresh spring water and diluted in a total volume of 25 litres to a final concentration of 1.6 × 10⁻⁵ g per litre. Suspensions were kept at 4°C and 1 litre samples were taken sterilely after 4, 11, 19, 27, 33 and 67 days. These samples were filtered, DNA was extracted and marker concentration was measured as described above.

### Example 3: Discrimination between human and ruminant faecal pollution in water

Water samples were taken from the following sampling sites:
- Cesspit (cesspit of an Austrian mountain hut with an average number of 100 residents during summer; sample taken in Summer 2005);
- River below WWTP (river water sample from a small river in central Austria right below the effluent pipe of a small waste water treatment plant of a small village, population equivalents around 500; sample taken in Winter 2005);
- River (water sample from pristine river in central Austria; samples taken in Winter 2005);
- Watering brook (sample taken from a small brook running through a remote fenced game feeding compound; sample taken in Winter 2005);
- Limestone Karst Spring Flood (sample from an alpine karst spring taken during a period of elevated flow conditions; sample taken in Summer 2005);
- Cattle Dung Heap Leachate (sample take from the seepage water of a dung heap on a cattle farm; sample taken in Winter 2005).

Water samples were taken and analysed according to the methods described above in Examples 1 and 2. Thereby, the qPCR analysis was performed in two separate reactions for the two markers (BacH and BacR) utilizing for each PCR reaction an aliquot of the same DNA extract of each sample.

The applicability of the described methods for discrimination of ruminant and human faecal pollution is demonstrated in Figure 4. The samples from the cesspit showed the highest concentration of human-specific marker measured in this experiment, while it lacked any detectable ruminant-specific marker. The sample from the river below the waste water treatment plant ("River below WWTP") contained 1000 times more human specific marker than the one above the plant. In contrast the numbers of ruminant-specific marker showed no change between those two sampling points on the river (i.e. "River" and "River below WWTP"). The sample from the remote watering brook did not exhibit any detectable human-specific marker while showing significant ruminant faecal markers. The karstic spring sampled during flood conditions ("Limestone Karst Spring Flood")contained much higher numbers of ruminant-specific marker than human-specific marker. Finally, the seepage water from the dung pile of a farm breeding cattle ("Cattle Dung Heap Leachate")contained high levels of ruminant-specific marker but no detectable human-specific marker.

This example demonstrates the applicability of the described methods for discrimination of faecal pollution from human and ruminant sources. The range of detectable pollution is spanned from very low levels, where classical faecal indicator parameters are unable to detect the pollution at all to levels comparable to concentrations encountered in faecal material.

### REFERENCES

Afonina, I., Zivarts, M., Kutyavin, I., Lukhtanov, E., Gamper, H., and Meyer, R.B: (1997) Efficient priming of PCR with short oligonucleotides conjugated to a minor groove binder. Nucleic Acids Research 25: 2657-2660.
Allsop, K., and Stickler, D.J. (1984) The enumeration of Bacteroides fragilis group organisms from sewage and natural waters. J Appl Bacteriol 56: 15-24.
Allsop, K., and Stickler, D.J. (1985) An assessment of Bacteroides fragilis group organisms as indicators of human faecal pollution. J Appl Bacteriol 58: 95-99.
Bernhard, A.E., and Field, K.G. (2000a) Identification of nonpoint sources of fecal pollution in coastal waters by using host-specific 16S ribosomal DNA genetic markers from fecal anaerobes. Appl Environ Microbiol 66: 1587-1594.
Bernhard, A.E., and Field, K.G. (2000b) A PCR assay to discriminate human and ruminant feces on the basis of host differences in Bacteroides-Prevotella genes encoding 16S rRNA. Appl Environ Microbiol 66: 4571-4574.
Bernhard, A.E., Goyard, T., Simonich, M.T., and Field, K.G. (2003) Application of a rapid method for identifying fecal pollution sources in a multi-use estuary. Water Res 37: 909-913.
BMGF, A.f.m.f.h.a.w.s.a. (2001) Trinkwasserverordnung: Verordnung über die Qualität von Wasser für den menschlichen Gebrauch (TWV). In Bundesgesetzblatt für die Republik Österreich*.*
Boehm, A.B., Fuhrman, J.A., Mrse, R.D., and Grant, S.B. (2003) Tiered approach for identification of a human fecal pollution source at a recreational beach: case study at Avalon Bay, Catalina Island, California. Environ Sci Technol 37: 673-680.
Brinkman, N.E., Haugland, R.A., Wymer, L.J., Byappanahalli, M., Whitman, R.L., and Vesper, S.J. (2003) Evaluation of a rapid, quantitative real-time PCR method for enumeration of pathogenic Candida cells in water. Appl Environ Microbiol 69: 1775-1782.
Bustin, S.A. (2000) Absolute quantification of mRNA using real-time reverse transcription polymerase chain reaction assays. J Mol Endocrinol 25: 169-193.
Dick, L.K., and Field, K.G. (2004) Rapid estimation of numbers of fecal Bacteroidetes by use of a quantitative PCR assay for 16S rRNA genes. Appl Environ Microbiol 70: 5695-5697.
Dick, L.K., Bernhard, A.E., Brodeur, T.J., Santo Domingo, J.W., Simpson, J.M., Walters, S.P., and Field, K.G. (2005) Host distributions of uncultivated fecal Bacteroidales bacteria reveal genetic markers for fecal source identification. Appl Environ Microbiol 71: 3184-3191.
Farnleitner, A.H., Mach, R.L., Burtscher, M.M., Reischer, G.H., Ryzinska, G., Keiblinger, K. et al. (2005) "Back to the roots" - incidence and abundance of faecal indicators in faeces and sewage of potential pollution sources in an alpine karstic catchment area. In IWA - Health related water microbiology symposium. Swansea, Wales.
Famleitner, A.H., Wilhartitz, I, Ryzinska, G., Kirschner, A.K., Stadler, H., Burtscher, M.M. et al. (2005) Bacterial dynamics in spring water of alpine karst aquifers indicates the presence of stable autochthonous microbial endokarst communities. Environ Microbiol 7: 1248-1259.
Fiksdal, L., Maki, J.S., LaCroix, S.J., and Staley, J.T. (1985) Survival and detection of Bacteroides spp., prospective indicator bacteria. Appl Environ Microbiol 49: 148-150.
Fogarty, L.R., and Voytek, M.A. (2005) Comparison of bacteroides-prevotella 16S rRNA genetic markers for fecal samples from different animal species. Appl Environ Microbiol 71: 5999-6007.
Ford, D.G., and Williams, P.W. (1996) Karst Geomorphology and Hydrology. London, UK: Chapman & Hall.
Giglio, S., P. T. Monis, and C. P. Saint. 2003. Demonstration of preferential binding of SYBR Green I to specific DNA fragments in real-time multiplex PCR. Nucleic Acids Res 31:e136.
Gordon, D.M. (2001) Geographical structure and host specificity in bacteria and the implications for tracing the source of coliform contamination. Microbiology 147: 1079-1085.
Griffiths, R.I., Whiteley, A.S., O'Donnell, A.G., and Bailey, M.J. (2000) Rapid method for coextraction of DNA and RNA from natural environments for analysis of ribosomal DNA- and rRNA-based microbial community composition. Appl Environ Microbiol 66: 5488-5491:
Guy, R.A., Payment, P., Krull, U.J., and Horgen, P.A. (2003) Real-time PCR for quantification of Giardia and Cryptosporidium in environmental water samples and sewage. Appl Environ Microbiol 69: 5178-5185.
Kralik, M. (2001) Strategie zum Schutz der Karstwassergebiete in Österreich. Vienna: Austrian environmental protection agency. Ist these Referenz ein Buch?
Kreader, C.A. (1995) Design and evaluation of Bacteroides DNA probes for the specific detection of human fecal pollution. Appl Environ Microbiol 61:1171-1179.
Kreader, C.A. (1998) Persistence of PCR-detectable Bacteroides distasonis from human feces in river water. Appl Environ Microbiol 64: 4103-4105.
Kutyavin, I.V., Afonina, I.A., Mills, A., Gorn, V.V., Lukhtanov, E.A., Belousov, E.S. et al. (2000) 3'-minor groove binder-DNA probes increase sequence specificity at PCR extension temperatures. Nucleic Acids Res 28: 655-661.
Letertre, C., Perelle, S., Dilasser, F., Arar, K. and Fach, P. (2003) Evaluation of the performance of LNA and MGB probes in 5 '-nuclease PCR assays. Molecular and Cellular Probes 17, 307-311.
Lorenz, R.J. (1992) Grundbegriffe der Biometrie. Jena, Germany: Gustav Fischer Verlag Stuttgart.
Kuhn, H., Demidov, V.V., Coull, J.M., Fiandaca, M.J., Gildea, B.D. and Frank-Kamenetskii, M.D. (2002) Hybridization of DNA and PNA molecular beacons to single-stranded and double-stranded DNA targets. JAm Chem Soc 124, 1097-1103.
Mackay, I.M., Arden, K.E., and Nitsche, A. (2002) Real-time PCR in virology. Nucleic Acids Res 30: 1292-1305.
Marras, S.A., Tyagi, S., and Kramer, F.R. (2006) Real-time assays with molecular beacons and other fluorescent nucleic acid hybridization probes. Clin Chim Acta 363: 48-60.
McCartney, H. A., S. J. Foster, B. A. Fraaije, and E. Ward. 2003. Molecular diagnostics for fungal plant pathogens. Pest Manag Sci 59:129-42.
Reischer, G.H., Lemmens, M., Farnleitner, A., Adler, A., and Mach, R.L. (2004) Quantification of Fusarium graminearum in infected wheat by species specific real-time PCR applying a TaqMan Probe. J Microbiol Methods 59: 141-146.
Roe, J.D., Haugland, R.A., Vesper, S.J., and Wymer, L.J. (2001) Quantification of Stachybotrys chartarum conidia in indoor dust using real time, fluorescent probe-based detection of PCR products. J Expo Anal Environ Epidemiol 11: 12-20.
Rousselon N., Delgenès J.P., and Godon J.J. (2004) A new real time PCR (TaqMan PCR) system for detection of the 16S rDNA gene associated with fecal bacteria. J Microbiol Methods 59(1):15-22
Savill, M.G., Murray, S.R., Scholes, P., Maas, E.W., McCormick, R.E., Moore, E.B., and Gilpin, B.J. (2001) Application of polymerase chain reaction (PCR) and TaqMan PCR techniques to the detection and identification of Rhodococcus coprophilus in faecal samples. J Microbiol Methods 47: 355-368.
Scott, T. M., J. B. Rose, T. M. Jenkins, S. R. Farrah, and J. Lukasik. 2002. Microbial source tracking: current methodology and future directions. Appl. Environ. Microbiol. 68:5796-803.
Seurinck, S., Defoirdt, T., Verstraete, W., and Siciliano, S.D. (2005) Detection and quantification of the human-specific HF 183 Bacteroides 16S rRNA genetic marker with real-time PCR for assessment of human faecal pollution in freshwater. Environ Microbiol 7: 249-259.
Simpson, J.M., Santo Domingo, J.W., and Reasoner, D.J. (2002) Microbial source tracking: state of the science. Environ Sci Technol 36: 5279-5288.
Sinton, L.W., Finlay, R.K., and Hannah, D.J. (1998) Distinguishing human from faecal contamination in water: a review. N Z J Mar Freshwater Res 32: 323-348.
Stults, J.R., Snoeyenbos-West, O., Methe, B., Lovley, D.R., and Chandler, D.P. (2001) Application of the 5' fluorogenic exonuclease assay (TaqMan) for quantitative ribosomal DNA and rRNA analysis in sediments. Appl Environ Microbiol 67: 2781-2789.
Suau, A., Bonnet, R., Sutren, M., Godon, J.J., Gibson, G.R., Collins, M.D., and Dore, J. (1999) Direct analysis of genes encoding 16S rRNA from complex communities reveals many novel molecular species within the human gut. Appl Environ Microbiol 65: 4799-4807.
Suzuki, M.T., Taylor, L.T., and DeLong, E.F. (2000) Quantitative analysis of small-subunit rRNA genes in mixed microbial populations via 5'-nuclease assays. Appl Environ Microbiol 66: 4605-4614.
Teske, A., Wawer, C., Muyzer, G., and Ramsing, N.B. (1996) Distribution of sulfate-reducing bacteria in a stratified fjord (Mariager Fjord, Denmark) as evaluated by most-probable-number counts and denaturing gradient gel electrophoresis of PCR-amplified ribosomal DNA fragments. Appl Environ Microbiol 62: 1405-1415.
WHO (2004) Guidelines for Drinking-water Quality. Geneva: World Health Organisation. (http:Hwww.who.int/water_sanitation_health/dwq/guidelines/en/)

## Claims

1. Oligonucleotide comprising a nucleic acid sequence selected from the group consisting of
TCATGATCCCATCCTG (SEQ ID NO: 1),
TCATGATGCCATCTTG (SEQ ID NO: 2),
TCATGATGCCATCCTG (SEQ ID NO: 8)
or the complementary nucleic acid sequences thereto.

2. The oligonucleotide according to claim 1, further comprising a minor groove binder (MGB), a locked nucleic acid (LNA) and/or a peptide nucleic acid (PNA).
and/or
further comprising at least one, preferably two detectable molecules,
wherein the detectable molecule is preferably selected from a chromophore, a fluorophore, a chemiluminescent molecule, an isotope, an enzyme, a coenzyme, an antigen, an antibody or antibody fragment, or a substrate.

3. The oligonucleotide according to claim 2, comprising two detectable molecules, wherein one is a fluorescence donor or reporter and the other is a fluorescence quencher or acceptor,
wherein the fluorescence donor or reporter is preferably selected from FAM, VIC, JOE, HEX, TET, Cy4, Texas Red, ROX, and the fluorescence quencher or acceptor is selected from NFQ, TAMRA, BodyPi 564/570, Cy5, Black Hole Quencher 1 (BHQ-1), BHQ-2, BHQ-3,
wherein more preferably the fluorescence donor or reporter is FAM and the fluorescence quencher or acceptor is NFQ.

4. Use of at least one oligonucleotide according to any of claims 1 to 3 as a probe for the detection and/or quantification of faecal pollution in an environmental sample, preferably comprising a quantitative real time PCR.

5. Use of at least one oligonucleotide according to any of claims 1 to 3 as a primer for the detection and/or quantification of faecal pollution in an environmental sample, preferably together with at least one oligonucleotide comprising a nucleic acid sequence selected from SEQ ID NOs: 3 to 7 or a nucleic acid sequence complementary thereto, preferably comprising a quantitative real time PCR.

6. The use according to claim 4 or 5, wherein said faecal pollution is human or ruminant faecal pollution,
preferably for discriminating between human and ruminant faecal pollution.
and/or
wherein the environmental sample is selected from a water sample, a soil or sediment sample, a compost sample, a biofilm sample, plant material, a meat sample, or a food product sample.

7. Composition comprising at least one oligonucleotide according to any of claims 1 to 3.

8. Kit for the detection and/or quantification of faecal pollution in an environmental sample by real time PCR comprising at least one oligonucleotide according to any of claims 1 to 3,
preferably further comprising primer designed by 16S rRNA gene sequence alignment, wherein more preferably at least one oligonucleotide is suitable to be used as a primer and at least one oligonucleotide is suitable to be used as a probe.

9. The kit according to claim 8, wherein the oligonucleotide suitable to be used as a probe comprises a nucleic acid sequence selected from SEQ ID NOs: 1, 2 or 8 or a nucleic acid sequence complementary thereto, preferably further comprising a quantification standard.

10. The kit according to claim 8 or 9, wherein said faecal pollution is human or ruminant faecal pollution,
preferably suitable for discriminating between human and ruminant faecal pollution.

11. The kit according to any of claims 8 to 10, wherein the environmental sample is selected from a water sample a soil or sediment sample, a compost sample, a biofilm sample, plant material, a meat sample, or a food product sample.

12. Method for the detection and/or quantification of faecal pollution in an environmental sample by detecting and/or quantifying at least one marker sequence, comprising the following steps
a) providing an environmental sample,
b) extracting nucleic acid from said sample,
c) subjecting the extracted nucleic acid to real time PCR using at least two oligonucleotides according to claims 1 to 3, wherein one is used as a primer and the other is used as a probe, or using a composition according to claim 7, and optionally, using a quantification standard with a defined amount of marker equivalents, and
d) optionally, quantifying the marker equivalents of the sample.

13. The method according to claim 12, wherein the at least one oligonucleotide used as a probe comprises a nucleic acid sequence selected from SEQ ID NOs: 1, 2 or 8 or a nucleic acid sequence complementary thereto.

14. The method according to claim 12 or 13, wherein said faecal pollution is human or ruminant faecal pollution,
preferably further comprising a discrimination between human and ruminant faecal pollution, wherein step c) more preferably comprises a second real time PCR.
and/or
wherein the environmental sample is selected from a water sample, a soil or sediment sample, a compost sample, a biofilm sample, plant material, a meat sample, or a food product sample.

15. Method for the identification of a microorganism as originating from human and/or ruminant faeces in a sample, comprising a real time PCR using at least two oligonucleotides according to claims 1 to 3, wherein one is used as a primer and the other is used as a probe.

## Patentansprüche

1. Oligonukleotid, umfassend eine Nukleinsäuresequenz, ausgewählt aus der Gruppe bestehend aus
TCATGATCCCATCCTG (SEQ ID Nr. 1),
TCATGATGCCATCTTG (SEQ ID Nr. 2),
TCATGATGCCATCCTG (SEQ ID Nr. 8)
oder den dazu komplementären Nukleinsäuresequenzen.

2. Oligonukleotid nach Anspruch 1, weiter umfassend einen kleine Furche-Binder (MGB), eine "locked" Nukleinsäure (LNA) und/oder eine Peptidnukleinsäure (PNA).
und/oder
weiter umfassend mindestens eines, bevorzugt zwei, nachweisbare Moleküle,
wobei das nachweisbare Molekül bevorzugt ausgewählt ist aus einem Chromophor, einem Fluorophor, einem chemilumineszenten Molekül, einem Isotop, einem Enzym, einem Coenzym, einem Antigen, einem Antikörper oder Antikörperfragment, oder einem Substrat.

3. Oligonukleotid nach Anspruch 2, umfassend zwei, nachweisbare Moleküle, wobei eines ein Fluoreszenz-Donor oder -reporter ist und das andere ein Fluoreszenz-Quencher oder -Akzeptor ist,
wobei Fluoreszenz-Donor oder -reporter bevorzugt ausgewählt ist aus FAM, VIC, JOE, HEX, TET, Cy4, Texas Red, ROX, und Fluoreszenz-Quencher oder -Akzeptor ausgewählt ist aus NFQ, TAMRA, BodyPi 564/570, Cy5, Black Hole Quencher 1 (BHQ-1), BHQ-2, BHQ-3,
wobei weiter bevorzugt der Fluoreszenz-Donor oder -reporter FAM ist, und der Fluoreszenz-Quencher oder -Akzeptor NFQ ist.

4. Verwendung von mindestens einem Oligonukleotid nach einem der Ansprüche 1 bis 3 als eine Sonde zum Nachweis und/oder der Quantifizierung von fäkaler Verunreinigung in einer Umwelt-Probe,
bevorzugt umfassend eine quantitative real-time PCR.

5. Verwendung von mindestens einem Oligonukleotid nach einem der Ansprüche 1 bis 3 als ein Primer zum Nachweis und/oder der Quantifizierung von fäkaler Verunreinigung in einer Umwelt-Probe,
bevorzugt zusammen mit mindestens einem Oligonukleotid umfassend eine Nukleinsäuresequenz, ausgewählt aus SEQ ID Nrn: 3 bis 7 oder eine dazu komplementäre Nukleinsäuresequenz,
bevorzugt umfassend eine quantitative real-time PCR.

6. Verwendung nach Anspruch 4 oder 5, wobei die fäkale Verunreinigung eine menschliche oder fäkale Verunreinigung durch Wiederkäuer ist,
bevorzugt zur Unterscheidung zwischen menschlicher und Wiederkäuer-fäkaler Verunreinigung,
und/oder
wobei die Umwelt-Probe ausgewählt ist aus einer Wasserprobe, einer Boden- oder Sedimentprobe, einer Kompostprobe, einer Biofilmprobe, Pflanzenmaterial, einer Fleischprobe oder einer Nahrungsmittelproduktprobe.

7. Zusammensetzung umfassend mindestens einem Oligonukleotid nach einem der Ansprüche 1 bis 3.

8. Kit zum Nachweis und/oder der Quantifizierung von fäkaler Verunreinigung in einer Umwelt-Probe durch real time PCR umfassend mindestens einem Oligonukleotid nach einem der Ansprüche 1 bis 3,
bevorzugt weiter umfassend Primer, die mittel 16S rRNA Gensequenzvergleich gestaltet werden,
wobei weiter bevorzugt mindestens ein Oligonukleotid zur Verwendung als ein Primer geeignet ist, und mindestens ein Oligonukleotid zur Verwendung als eine Sonde geeignet ist.

9. Kit nach Anspruch 8, wobei das zur Verwendung als eine Sonde geeignete Oligonukleotid eine Nukleinsäuresequenz ausgewählt aus SEQ ID Nm: 1, 2 oder 8 oder eine dazu komplementäre Nukleinsäuresequenz umfasst, bevorzugt weiter umfassend einen Quantifizierungsstandard.

10. Kit nach Anspruch 8 oder 9, wobei die fäkale Verunreinigung eine menschliche oder fäkale Verunreinigung durch Wiederkäuer ist,
bevorzugt geeignet zur Unterscheidung zwischen menschlicher und Wiederkäuer-fäkaler Verunreinigung.

11. Kit nach einem der Ansprüche 8 bis 10, wobei die Umwelt-Probe ausgewählt ist aus einer Wasserprobe, einer Boden- oder Sedimentprobe, einer Kompostprobe, einer Biofilmprobe, Pflanzenmaterial, einer Fleischprobe oder einer Nahrungsmittelproduktprobe.

12. Verfahren zum Nachweis und/oder der Quantifizierung von fäkaler Verunreinigung in einer Umwelt-Probe durch Nachweisen und/oder Quantifizieren von mindestens einer Markersequenz, umfassend die folgenden Schritte
a) Zur Verfügung stellen einer Umwelt-Probe,
b) Extrahieren von Nukleinsäure aus der Probe,
c) Unterziehen der extrahierten Nukleinsäure einer real time PCR unter der Verwendung von mindestens zwei Oligonukleotiden nach den Ansprüchen 1 bis 3, wobei eines als ein Primer verwendet wird und das andere als eine Sonde verwendet wird, oder unter der Verwendung eine Zusammensetzug nach Anspruch 7, und gegebenenfalls, unter der Verwendung eines Quantifizierungsstandards mit einer definierten Menge an Markeräquivalenten, und
d) gegebenenfalls, Quantifizieren der Markeräquivalente der Probe.

13. Verfahren nach Anspruch 12, wobei das als eine Sonde verwendete mindestens eine Oligonukleotid eine Nukleinsäuresequenz ausgewählt aus SEQ ID Nm: 1, 2 oder 8, oder eine dazu komplementäre Nukleinsäuresequenz umfasst.

14. Verfahren nach Anspruch 12 oder 13, wobei die fäkale Verunreinigung menschliche oder Wiederkäuer-fäkale Verunreinigung ist,
bevorzugt weiter umfassend eine Unterscheidung zwischen menschlicher oder Wiederkäuer-fäkaler Verunreinigung, wobei Schritt c) weiter bevorzugt eine zweite real time-PCR umfasst.
und/oder
wobei die Umwelt-Probe ausgewählt ist aus einer Wasserprobe, einer Boden- oder Sedimentprobe, einer Kompostprobe, einer Biofilmprobe, Pflanzenmaterial, einer Fleischprobe oder einer Nahrungsmittelproduktprobe.

15. Verfahren zur Identifizierung eines Mikroorganismus als aus menschlichem und/oder Wiederkäuer-Fäzes abstammend in einer Probe, umfassend eine real time-PCR unter der Verwendung von mindestens zwei Oligonukleotiden nach den Ansprüchen 1 bis 3, wobei eines als ein Primer verwendet wird und das andere als eine Sonde verwendet wird.

## Revendications

1. Oligonucléotide comprenant une séquence d'acides nucléiques choisie dans le groupe constitué des séquences :
TCATGATCCCATCCTG (SEQ ID NO: 1),
TCATGATGCCATCTTG (SEQ ID NO: 2),
TCATGATGCCATCCTG (SEQ ID NO: 8)
ou des séquences d'acides nucléiques qui lui sont complémentaires.

2. Oligonucléotide selon la revendication 1, comprenant en outre un liant de petit sillon (MGB), un acide nucléique bloqué (LNA) et/ou un acide nucléique peptidique (PNA).
et/ou
comprenant en outre au moins une molécule détectable, de préférence deux, où la molécule détectable est de préférence choisie parmi un chromophore, un fluorophore, une molécule chimiluminescente, un isotope, une enzyme, une coenzyme, un antigène, un anticorps ou un fragment d'anticorps, ou un substrat.

3. Oligonucléotide selon la revendication 2, comprenant deux molécules détectables, dans lequel l'une des molécules est un donneur ou un rapporteur de fluorescence et l'autre molécule est un accepteur ou un désactiveur de fluorescence,
dans lequel le donneur ou le rapporteur de fluorescence est de préférence choisi parmi FAM, VIC, JOE, HEX, TET, Cy4, Texas Red, ROX, et l'accepteur ou le désactiveur de fluorescence est choisi parmi NFQ, TAMARA, BodyPi 564/570, Cy5, Quencher Black Hole 1 (BHQ-1), BHQ-2, BHQ-3,
où plus préférablement le donneur ou le rapporteur de fluorescence est FAM et l'accepteur ou le désactiveur de fluorescence est NFQ.

4. Utilisation d'au moins un oligonucléotide selon l'une des revendications 1 à 3 comme sonde pour détecter et/ou quantifier la pollution fécale dans un échantillon environnemental, comprenant de préférence une PCR quantitative en temps réel.

5. Utilisation d'au moins un oligonucléotide selon l'une des revendications 1 à 3 comme amorce pour détecter et/ou quantifier la pollution fécale dans un échantillon environnemental, de préférence avec au moins un oligonucléotide comprenant une séquence d'acides nucléiques choisie parmi les séquences SEQ ID NO: 3 à 7 ou une séquence d'acides nucléiques qui lui est complémentaire, comprenant de préférence une PCR quantitative en temps réel.

6. Utilisation selon la revendication 4 ou 5, dans laquelle ladite pollution fécale est une pollution fécale humaine ou de ruminants,
de préférence pour distinguer entre la pollution fécale humaine et celle des ruminants, et/ou
dans laquelle l'échantillon environnemental est choisi parmi un échantillon d'eau, un échantillon de sol ou de sédiment, un échantillon de compost, un échantillon de biofilm, un matériau végétal, un échantillon de viande, ou un échantillon de produit alimentaire.

7. Composition comprenant au moins un oligonucléotide selon l'une des revendications 1 à 3.

8. Kit pour la détection et/ou la quantification de la pollution fécale dans un échantillon environnemental par PCR en temps réel comprenant au moins un oligonucléotide selon l'une des revendications 1 à 3,
de préférence comprenant en outre une amorce conçue par un alignement de séquences du gène 16S rRNA, où plus préférablement au moins un oligonucléotide que l'on peut utiliser comme amorce et au moins un oligonucléotide que l'on peut utiliser comme sonde.

9. Kit selon la revendication 8, dans lequel l'oligonucléotide que l'on peut utiliser comme sonde comprend une séquence d'acides nucléiques choisie parmi les séquences SEQ ID NO: 1, 2 ou 8 ou une séquence d'acides nucléiques qui lui est complémentaire, de préférence comprenant en outre une référence de quantification.

10. Kit selon la revendication 8 ou 9, dans lequel ladite pollution fécale est une pollution fécale humaine ou de ruminants,
de préférence adapté pour distinguer entre la pollution fécale humaine et la pollution fécale de ruminants.

11. Kit selon l'une des revendications 8 à 10, dans lequel l'échantillon environnemental est choisi parmi un échantillon d'eau, un échantillon de sol ou de sédiment, un échantillon de compost, un échantillon de biofilm, un matériau végétal, un échantillon de viande, ou un échantillon de produit alimentaire.

12. Procédé destiné à détecter et/ou à quantifier la pollution fécale dans un échantillon environnemental en détectant et/ou en quantifiant au moins une séquence de marqueur, comprenant les étapes qui consistent
a) à fournir un échantillon environnemental,
b) à extraire un acide nucléique à partir dudit échantillon,
c) à soumettre l'acide nucléique extrait à une PCR en temps réel en utilisant au moins deux oligonucléotides selon les revendications 1 à 3, où l'un est utilisé comme amorce et l'autre est utilisé comme sonde, ou en utilisant une composition selon la revendication 7, et éventuellement, en utilisant une référence de quantification avec une quantité définie d'équivalents de marqueur, et
d) éventuellement, à quantifier des équivalents de marqueur de l'échantillon.

13. Procédé selon la revendication 12, dans lequel l'au moins un oligonucléotide utilisé comme sonde comprend une séquence d'acides nucléiques choisie parmi les séquences SEQ ID NO: 1, 2 ou 8 ou une séquence d'acides nucléiques qui lui est complémentaire.

14. Procédé selon la revendication 12 ou 13, dans lequel ladite pollution fécale est une pollution fécale humaine ou de ruminants,
de préférence comprenant en outre une distinction entre la pollution fécale humaine et celle de ruminants, dans lequel l'étape c) comprend plus préférablement une deuxième PCR en temps réel,
et/ou
dans lequel l'échantillon environnemental est choisi parmi un échantillon d'eau, un échantillon de sol ou de sédiment, un échantillon de compost, un échantillon de biofilm, un matériau végétal, un échantillon de viande, ou un échantillon de produit alimentaire.

15. Procédé d'identification d'un microorganisme provenant de matières fécales humaines et/ou de ruminants dans un échantillon, comprenant une PCR en temps réel en utilisant au moins deux oligonucléotides selon les revendications 1 à 3, dans lequel l'un est utilisé comme amorce et l'autre est utilisé comme sonde.


### SEQUENCE LISTING

<110> Technische Universität Wien
<120> Detection and quantification of faecal pollution in an environmental sample
<130> T30558PCT
<160> 8
<170> PatentIn version 3.2
<210> 1
   <211> 16
   <212> DNA
   <213> Bacteroidetes
<400> 1
   tcatgatccc atcctg 16
<210> 2
   <211> 16
   <212> DNA
   <213> Bacteroidetes
<400> 2
   tcatgatgcc atcttg 16
<210> 3
   <211> 17
   <212> DNA
   <213> Bacteroidetes
<400> 3
   cttccgaaag ggagatt 17
<210> 4
   <211> 20
   <212> DNA
   <213> Bacteroidetes
<400> 4
   cttggccagc cttctgaaag 20
<210> 5
   <211> 18
   <212> DNA
   <213> Bacteroidetes
<400> 5
   atcgtctacc gaaaatac 18
<210> 6
   <211> 18
   <212> DNA
   <213> Bacteroidetes
<400> 6
   gcgtatccaa ccttcccg 18
<210> 7
   <211> 17
   <212> DNA
   <213> Bacteroidetes
<400> 7
   atccccatcc gttaccg 17
<210> 8
   <211> 16
   <212> DNA
   <213> Bacteroidetes
<400> 8
   tcatgatgcc atcctg 16